(19) Europäisches Patentamt
European Patent Office
Office européen des brevets

(11) EP 4 393 930 B1

(12) **EUROPEAN PATENT SPECIFICATION**

(45) Date of publication and mention of the grant of the patent:
**29.07.2026 Bulletin 2026/31**

(21) Application number: **22860510.1**

(22) Date of filing: **23.08.2022**

(51) International Patent Classification (IPC):
***C07F 9/6558*** *(2006.01)* ***C07D 401/12*** *(2006.01)*
***A61P 35/00*** *(2006.01)* ***A61P 35/02*** *(2006.01)*
***A61K 45/06*** *(2006.01)* ***A61K 31/675*** *(2006.01)*
***A61K 31/662*** *(2006.01)*

(52) Cooperative Patent Classification (CPC):
(C-Sets available)
**C07D 401/12; A61K 31/662; A61K 31/675; A61K 45/06; A61P 35/00; A61P 35/02; C07F 9/65583** (Cont.)

(86) International application number:
**PCT/CN2022/114340**

(87) International publication number:
**WO 2023/025164 (02.03.2023 Gazette 2023/09)**

(54) **CRYSTAL FORM AND APPLICATION OF ARYL PHOSPHINE OXIDE COMPOUND**

KRISTALLFORM UND ANWENDUNG EINER ARYLPHOSPHINOXIDVERBINDUNG

FORME CRISTALLINE ET APPLICATION D'UN COMPOSÉ D'OXYDE D'ARYLE PHOSPHINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2021 CN 202110995154**

(43) Date of publication of application:
**03.07.2024 Bulletin 2024/27**

(73) Proprietor: **Chengdu Di'Ao Jiuhong Pharmaceutical Factory**
**Chengdu, Sichuan 610041 (CN)**

(72) Inventors:
- **HE, Peng**
**Chengdu, Sichuan 610041 (CN)**
- **LI, Bogang**
**Chengdu, Sichuan 610041 (CN)**
- **YU, Zhou**
**Chengdu, Sichuan 610041 (CN)**
- **DONG, Guangxin**
**Chengdu, Sichuan 610041 (CN)**
- **LI, Haiyan**
**Chengdu, Sichuan 610041 (CN)**
- **DENG, Ta**
**Chengdu, Sichuan 610041 (CN)**
- **ZHANG, Rui**
**Chengdu, Sichuan 610041 (CN)**
- **HUANG, Pei**
**Chengdu, Sichuan 610041 (CN)**

(74) Representative: **Kador & Partner Part mbB**
**Corneliusstraße 15**
**80469 München (DE)**

(56) References cited:
**WO-A1-2020/147838**
**WO-A1-2021/018003**
**WO-A1-2021/057882**
**WO-A1-2021/073498**
**WO-A1-2022/002241**
**WO-A1-2022/002241**
**CN-A- 102 105 150**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/675, A61K 2300/00**

## Description

### TECHNICAL FIELD

The present disclosure relates to the field of medical chemistry, in particular to a medicinal aryl phosphorus oxide compound, dihydrate crystal form thereof, preparation method and use thereof.

### BACKGROUNG

Protein kinases represent a large family of proteins that play an important role in the regulation of a plurality of cellular processes and in the maintenance and control of cell functions, which include proliferation, apoptosis, cytoskeletal rearrangement, differentiation, development, immunoreaction, nervous system function and conduction. In addition, many diseases and/or functional disorders are associated with aberrant, abnormal or deregulated activity of one or more kinases.

Lung cancer is one of the most common malignant tumors, generally divided into Small Cell Lung Cancer (SCLC) and Non-Small Cell Lung Cancer (NSCLC), and the lung cancer ranks first whether in China or the world. The Non-Small Cell Lung Cancer (NSCLC) accounts for more than 80% of all lung cancers, which seriously threatens human health (Chinese Journal of Lung Cancer [J], 2012 Feb 20; 15(2): 106-111).

EGFR with a full name of epidermal growth factor receptor is a transmembrane glycoprotein with tyrosine kinase activity widely distributed on cell membranes of various human tissues. Mutation and abnormal activation of EGFR are closely related to the occurrence and development, grade malignancy, metastasis with various tumors such as non-small cell lung cancer, breast cancer, esophageal cancer. Most patients with Non-Small Cell Lung Cancer (NSCLC) have EGFR overexpression, and about 40-50% of Non-Small Cell Lung Cancer patients in Asia (especially in China) belong to EGFR mutations, so EGFR inhibition can significantly improve the survival time of the NSCLC patients. Common mutations of EGFR may be divided into two categories, one category refers to drug-sensitive mutations, i.e., anti-tumor targeted drugs may be used after mutation, such as deletions at exon 19, and L858R mutation at exon 21; while the other category refers to drug-resistant mutations, i.e., resistant to a certain anti-tumor targeted drug after mutation, such as T790M mutation, and C797S mutation. The first-generation EGFR small molecule inhibitor drugs Gefitinib, Erlotinib and Icotinib obtain remarkable clinical therapeutic effects in patients with EGFR-sensitive mutations, and prolong survival time. However, most patients who benefit from these drugs develop drug resistance after taking the drugs for several months. More than 50% of the drug-resistant patients develop drug resistance due to the T790M mutation in EGFR. The second-generation EGFR irreversible inhibitor drugs Afatinib and Neratinib obtain better results in preclinical research, but lack selectivity on wild-type EGFR (EGFRWT), and have large side effects such as skin toxicity. The third-generation irreversible inhibitor Osimertinib (AZD9291) overcomes the drug resistance of EGFR T790M, and can effectively treat advanced non-small cell lung cancer patients with epidermal growth factor receptor T790M mutation or drug resistance to other EGFR inhibitors in clinic. Although the Osimertinib has great success in clinically treating the non-small cell lung cancer with EGFR T790M mutation, part of the patients who benefit from the Osimertinib have drug resistance after 9-14 months of treatment (Nature Medicine 2015, 21(6), 560-562). It was found that in up to 40% of the drug-resistant patients, Osimertinib resistance was caused due to point mutation at (EGFR) C797S. Further mechanistic studies have shown that the point mutations at (EGFR) C797S convert cysteine at position 797 to serine, resulting in the inability of the Osimertinib to form covalent bonds with target proteins, ultimately leading to drug resistance. At present, there is no clinically available EGFR inhibitor that is effective against the new mutation (C797S). Therefore, a novel EGFR inhibitor with high selectivity is urgently needed to solve the problems of drug resistance caused by the point mutation at (EGFR) C797S.

Anaplastic lymphoma kinase (ALK), also known as ALK tyrosine kinase receptor or CD246, is an active enzyme encoded by ALK gene in human body. A fusion gene formed by ALK is closely related to the occurrence and development of various tumors such as non-small cell lung cancer. In the non-small cell lung cancer patients, fusion oncogenes such as EML4-ALK (fusion gene of microtubule-associated protein 4 and anaplastic lymphoma kinase in echinoderms) account for about 3-7%. Therefore, it is of great clinical value to develop protein kinase inhibitors to positive ALK fusion genes. On the other hand, in recent years, with the increase of non-small cell lung cancer patients and the popularization of the second-generation sequencing technology (deep sequencing), researchers have found that EGFR mutant and ALK gene fusion can occur simultaneously in some non-small cell lung cancer patients. Therefore, a novel protein kinase inhibitor with high selectivity is urgently needed to solve the problems of drug resistance caused by point mutation of (EGFR) C797S, and solve the fusion and mutation of the ALK genes at the same time.

WO 2020/147838 A1 discloses a salt of an EGFR inhibitor, a crystal form thereof, a preparation method therefor, and an application of the salt and the crystal form in the preparation of a treatment for non-small cell lung cancer.

SUMMARY

The invention is defined by the claims.

One or more embodiments provide a crystal of dihydrate of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide with crystal form 1, wherein using Cu-$K_\alpha$ radiation and shown at 20 angle, the X-ray powder diffraction pattern of the crystal form 1 has the following characteristic peaks: 5.54±0.2°, 11.06°±0.2, 19.12°±0.2 and 27.90±0.2°.

In one or more embodiments, using Cu-$K_\alpha$ radiation and shown at 2θ angle, the X-ray powder diffraction pattern of the crystal form 1 has the following characteristic peaks: 5.54±0.2°, 11.06±0.2°, 16.15±0.2°, 16.73:!:0.2°, 17.10±0.2°, 19.12±0.2°, 21.18±0.2°, 24.16±0.2°, 26.27±0.2°, 26.48±0.2°, 27.90±0.2° and 33.64±0.2°.

In one or more embodiments, using Cu-$K_\alpha$ radiation and shown at 2θ angle, the X-ray powder diffraction pattern of the crystal form 1 has the following characteristic peaks: 5.54±0.2°, 8.50±0.2°, 10.53±0.2°, 11.06±0.2°, 11.48±0.2°, 13.00±0.2°, 16.15±0.2°, 16.73±0.2°, 17.10±0.2°, 19.12±0.2°, 19.47± 0.2°, 21.18±0.2°, 24.16±0.2°, 26.27±0.2°, 26.48±0.2°, 27.90±0.2° and 33.64±0.2°.

In one or more embodiments, using Cu-$K_\alpha$ radiation and shown at 2θ angle, the X-ray powder diffraction pattern of the crystal form 1 has the following characteristic peaks: 5.54±0.2°, 8.50±0.2°, 10.53±0.2°, 11.06±0.2°, 11.48±0.2°, 13.00±0.2°, 16.15±0.2°, 16.73±0.2°, 17.10±0.2°, 18.34±0.2°, 19.12±0.2°, 19.47±0.2°, 21.18±0.2°, 22.21±0.2°, 22.65±0.2°, 23.10±0.2°, 23.49±0.2°, 24.16±0.2°, 26.27±0.2°, 26.48±0.2°, 26.93±0.2°, 27.35±0.2°, 27.90±0.2°, 29.35±0.2°, 32.22±0.2°, 33.64±0.2° and 34.17±0.2°。

In one or more embodiments, using Cu-$K_\alpha$ radiation and shown at 2θ angle, the X-ray powder diffraction pattern of the crystal form 1 is shown in Figure 1.

One or more embodiments provide an anhydrous crystal of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide with crystal form 2, wherein using Cu-$K_\alpha$ radiation and shown at 2θ angle, the X-ray powder diffraction pattern of the crystal form 2 has the following characteristic peaks: 5.72±0.2°, 10.24±0.2°, 11.44±0.2°, 15.07±0.2°, 15.09±0.2°, 15.92 ±0.2°, 16.41±0.2°, 17.07±0.2°, 17.38±0.2°, 18.49±0.2°, 19.49±0.2°, 20.07±0.2°, 21.59±0.2°, 22.41±0.2°, 23.25 ±0.2°, 23.55±0.2° and 25.19±0.2°.

In one or more embodiments, using Cu-$K_\alpha$ radiation and shown at 2θ angle, the X-ray powder diffraction pattern of the crystal form 2 has the following characteristic peaks: 5.72±0.2°, 10.24±0.2°, 11.44±0.2°, 15.07±0.2°, 15.09±0.2°, 15.92±0.2°, 16.41±0.2°, 17.07±0.2°, 17.38±0.2°, 18.49±0.2°, 18.71±0.2°, 19.49±0.2°, 20.07±0.2°, 21.59±0.2°, 22.41±0.2°, 23.05±0.2°, 23.25±0.2°, 23.55±0.2°, 24.71±0.2°, 25.19±0.2°, 26.36±0.2°, 27.22±0.2°, 27.51±0.2°, 28.45±0.2°, 29.49±0.2° and 29.95±0.2°.

In one or more embodiments, using Cu-$K_\alpha$ radiation and shown at 2θ angle, the X-ray powder diffraction pattern of the crystal form 2 is shown in Figure 2.

One or more embodiments provide an anhydrous crystal of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide with crystal form 3, wherein using Cu-$K_\alpha$ radiation and shown at 2θ angle, the X-ray powder diffraction pattern of the crystal form 3 has the following characteristic peaks: 4.86±0.2°, 5.62±0.2°, 6.88±0.2°, 8.16±0.2°, 10.54±0.2°, 12.31 ±0.2°, 15.90±0.2°, 16.22±0.2°, 17.21±0.2°, 18.30±0.2°, 19.73±0.2°, 20.67±0.2°, 21.43±0.2°, 22.97±0.2°, 23.69 ±0.2°, 25.51±0.2°, 28.77±0.2° and 30.39±0.2°.

In one or more embodiments, using Cu-$K_\alpha$ radiation and shown at 2θ angle, the X-ray powder diffraction pattern of the crystal form 3 has the following characteristic peaks: 4.86±0.2°, 5.62±0.2°, 6.88:!:0.2°, 8.16±0.2°, 10.54±0.2°, 12.31 ±0.2°, 14.32±0.2°, 15.90±0.2°, 16.22±0.2°, 17.21±0.2°, 18.30±0.2°, 19.73± 0.2°, 20.67±0.2°, 21.43±0.2°, 22.97 ±0.2°, 23.69±0.2°, 25.51±0.2°, 27.59±0.2°, 28.07:!:0.2°, 28.77±0.2°, 29.09±0.2°, 30.39±0.2°, 34.55±0.2° and 35.32 ±0.2°.

In one or more embodiments, using Cu-$K_\alpha$ radiation and shown at 2θ angle, the X-ray powder diffraction pattern of the crystal form 3 is shown in Figure 3.

One or more embodiments provide a pharmaceutical composition comprising the crystal of dihydrate with crystal form 1, the anhydrous crystal with crystal form 2 or the anhydrous crystal with crystal form 3 according to the present application, and a pharmaceutically acceptable carrier.

One or more embodiments provide a pharmaceutical composition comprising the crystal of dihydrate with crystal form 1, the anhydrous crystal with crystal form 2 or the anhydrous crystal with crystal form 3 according to the present application, and other anticancer drug or antitumor drug.

In one or more embodiments, the anticancer drug or antitumor drug is one or more of cytotoxic drug, hormone drug, antimetabolite drug, tumor-targeted drug, PARP inhibitor drug, adjuvant therapy drug or antitumor biological drug.

In one or more embodiments, the cytotoxic drug is one or more of carboplatin, cisplatin, irinotecan, paclitaxel, fluorouracil, cytarabine, lenalidomide, and tretinoin.

In one or more embodiments, the hormone drug is one or more of dexamethasone, fulvestrant, and tamoxifen.

In one or more embodiments, the antimetabolite drug is one or more of fluorouracil, methotrexate, furanofluorouracil, and cytarabine.
In one or more embodiments, the tumor-targeted drug is one or more of imatinib, erlotinib, and lapatinib.
In one or more embodiments, the PARP inhibitor drug is one or more of Olaparib, Rubraca, and Zejula.
In one or more embodiments, the adjuvant therapy drug is one or more of the recombinant human granulocyte colony-stimulating factor, erythropoietin, disodium pamidronate, and zoledronic acid.
In one or more embodiments, the antitumor biological drug is one or more of Keytruda, Opdivo, Tecentriq, Imfinzi, and Bavencio.
One or more embodiments provide a use of the pharmaceutical composition comprising the crystal of dihydrate with crystal form 1, the anhydrous crystal with crystal form 2 or the anhydrous crystal with crystal form 3 according to the present application in the preparation of a medicament for the prevention and/or treatment of cancer.
In one or more embodiments, the cancer is plasmacytoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophagus cancer, squamous cell carcinoma, or colon cancer.
One or more embodiments provide a use of the pharmaceutical composition comprising the crystal of dihydrate with crystal form 1, the anhydrous crystal with crystal form 2 or the anhydrous crystal with crystal form 3 according to the present application in the preparation of EGFR inhibitor or ALK inhibitor or EGFR and ALK inhibitor or protein kinase inhibitor.
In one or more embodiments, the EGFR inhibitor or ALK inhibitor or EGFR and ALK inhibitor or protein kinase inhibitor is used for the treatment of plasmacytoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophagus cancer, squamous cell carcinoma, or colon cancer.
In one or more embodiments, the EGFR has one or more mutations selected from the group consisting of L858R mutation, Del19 mutation, T790M mutation and C797S mutation.
In one or more embodiments, the EGFR has a C797S mutation.
In one or more embodiments, the ALK has EML-4-ALK fusion and/or EML-4-ALK-L1196M mutation.
One or more embodiments provide a method for the preparation of the crystal of dihydrate according to the present application, comprising slurrying the compound of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide in a mixture of ethyl acetate and water at 10 °C to 30 °C (e.g., 10 °C, 15 °C, 20 °C, 25 °C, or 30 °C), filtering, washing the filter cake with ethyl acetate, and then the vacuum drying the filter cake to 5 wt% to 6 wt% (based on the weight of the filter cake) of moisture to obtain a crystalline substance.
In one or more embodiments, the weight ratio of ethyl acetate to water is 5:1 to 10:1, such as 5:1, 6:1, 7:1, 8:1, 9: 1 or 10: 1.
In one or more embodiments, the slurrying time is 30 to 120 minutes (e.g., 30 minutes, 40 minutes, 50 minutes, 60 minutes, 70 minutes, 80 minutes, 90 minutes, 100 minutes, 110 minutes, or 120 minutes).
In one or more embodiments, the slurrying time is 50 to 70 minutes.
In one or more embodiments, vacuum drying at a temperature of 15 °C to 35 °C (15 °C, 20 °C, 25 °C, 30 °C, or 35 °C) and under a pressure of -0.05 to -0.1 MPa (e.g., -0.05 MPa, -0.06 MPa, -0.07 MPa, -0.08 MPa, -0.09 MPa, or -0.1 MPa).
In one or more embodiments, the vacuum drying is carried out at a temperature of 20 °C to 30 °C and under a pressure of -0.08 to -0.1 MPa.
In one or more embodiments, vacuum drying to 4% to 7% of moisture (e.g., 4%, 5%, 6%, or 7%).
In one or more embodiments, vacuum drying to 5% to 6% of moisture.
One or more embodiments provide a method for the preparation of the crystal of dihydrate according to the present application, comprising a volatile crystallization method, a slurry crystallization method, an antisolvent crystallization method, and a cooling crystallization method.
In one or more embodiments, in the volatile crystallization method, dissolving the compound of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide in the first solvent and/or the second solvent at a temperature of 10°C to 40 °C (e.g., 10 °C, 15 °C, 20 °C, 25 °C, 30 °C, 35 °C, or 40 °C), filtering, and evaporating the filtrate until dry to obtain a crystalline substance.
In one or more embodiments, in the volatile crystallization method, the temperature, the first solvent, the second solvent and the volume ratio thereof are as described in the following table:

| Temperature | The first solvent | The second solvent | Volume ratio of the first solvent to the second solvent |
|---|---|---|---|
| 40°C | Methanol | - | - |
| 10-30°C | Ethanol | - | - |
| 40°C | Isopropanol | - | - |
| 10-30°C | Acetone | - | - |
| 40°C | Ethyl acetate | - | - |
| 10-30°C | Tetrahydrofuran | - | - |
| 10-30°C | Chloroform | - | - |
| 10-30°C | Methanol | Water | 2.0/0.1 |
| 10-30°C | Ethanol | Water | 3.0/0.1 |
| 10-30°C | Acetone | Water | 5.0/0.1 |
| 10-30°C | Tetrahydrofuran | Water | 5.0/0.1 |
| 40°C | Methanol | Ethyl acetate | 2.0/1.0 |
| 10-30°C | Ethanol | n-Heptane | 3.0/1.0 |
| 10-30°C | Acetone | Methyl tert-butyl ether | 9.0/3.0 |
| 10-30°C | Butanone | Acetonitrile | 8.0/3.0 |
| 10-30°C | Tetrahydrofuran | n-Heptane | 5.0/2.0 |
| 40°C | Chloroform | Methylcyclohexane | 1.0/1.0 |

In one or more embodiments, in the slurry crystallization method, adding the compound of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide to the first solvent and/or the second solvent at a temperature of 4°C to 40 °C (4 °C, 5 °C, 6 °C, 7 °C, 8 °C, 9 °C, 10 °C, 15 °C, 20 °C, 25 °C, 30 °C, 35 °C, or 40 °C) to form a suspension, after slurrying the suspension for 1 to 3 days (such as 1, 2 or 3 days) , then centrifugating, and drying the solid under the condition of a temperature of 20 °C to 30 °C (20 °C, 21 °C, 22 °C, 23 °C, 24 °C, 25 °C, 26 °C, 27 °C, 28 °C, 29 °C, or 30 °C) and a pressure of -0.08 to -0.1MPa (such as -0.08 MPa, -0.09 MPa or -0.1MPa) to obtain a crystalline substance.

In one or more embodiments, in the slurry crystallization method, the temperature, the first solvent, the second solvent and the volume ratio thereof are as described in the following table:

| Temperature | The first solvent | The second solvent | Volume ratio of the first solvent to the second solvent |
|---|---|---|---|
| 4°C | Methanol | - | - |
| 10-30°C | Ethanol | - | - |
| 10-30°C | Water | - | - |
| 4°C | Acetone | - | - |
| 40°C | Isopropyl ether | - | - |
| 10-30°C | Ethyl acetate | - | - |
| 10-30°C | Methyl tert-butyl ether | - | - |
| 40°C | Isopropyl acetate | - | - |
| 4°C | Tetrahydrofuran | - | - |
| 10-30°C | Acetonitrile | - | - |
| 10-30°C | Toluene | - | - |
| 10-30°C | n-Heptane | - | - |
| 10-30°C | Methanol | Water | 1.0/1.0 |

(continued)

| Temperature | The first solvent | The second solvent | Volume ratio of the first solvent to the second solvent |
|---|---|---|---|
| 10-30°C | Ethanol | Water | 1.0/1.0 |
| 10-30°C | Acetone | Water | 1.5/0.5 |
| 10-30°C | Water saturated with ethyl acetate | | - |
| 4°C | Tetrahydrofuran | Water | 0.8/1.2 |
| 10-30°C | 1,4-Dioxane | Water | 0.7/1.3 |
| 40°C | Acetonitrile | Water | 1.4/0.6 |
| 10-30°C | Dimethyl sulfoxide | Water | 1.0/1.0 |
| 4°C | Ethanol | n-Heptane | 1.0/1.0 |
| 10-30°C | Isopropyl alcohol | Isopropyl ether | 1.0/1.0 |
| 10-30°C | Acetone | Methyl tert-butyl ether | 1.0/1.0 |
| 40°C | Butanone | Toluene | 1.0/1.0 |
| 10-30°C | Ethyl acetate | Methyl tert-butyl ether | 1.0/1.0 |
| 40°C | Isopropyl acetate | Methylcyclohexane | 1.0/1.0 |
| 10-30°C | Tetrahydrofuran | n-Heptane | 0.5/1.5 |
| 10-30°C | 1,4-Dioxane | Isopropyl ether | 0.5/1.5 |
| 10-30°C | Acetonitrile | Butyl acetate | 1.0/1.0 |
| 40°C | Acetonitrile | Methyl tert-butyl ether | 0.8/1.2 |
| 10-30°C | Chloroform | Methylcyclohexane | 0.5/1.5 |

In one or more embodiments, in the antisolvent crystallization method, dissolving the compound of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide in the first solvent and then adding to the second solvent at a temperature of 10 °C to 30 °C (such as 10 °C, 15 °C, 20 °C, 25 °C, or 30 °C), or after dissolving the compound in the first solvent, then adding the second solvent to the first solvent, precipitating the solid, stirring, centrifugating, and drying the solid at a temperature of 20 °C to 30 °C (such as 20 °C, 21 °C, 22 °C, 23 °C, 24 °C, 25 °C, 26 °C, 27 °C, 28 °C, 29 °C, or 30 °C) and under a pressure of -0.08 to -0.1MPa (such as -0.08 MPa, -0.09 MPa or -0.1 MPa) to obtain a crystalline substance.

In one or more embodiments, in the antisolvent crystallization method, the first solvent, the second solvent and the volume ratio thereof are as described in the following table:

| The first solvent | The second solvent | Volume ratio of the first solvent to the second solvent | Addition manner |
|---|---|---|---|
| Methanol | Water | 2.0/3.0 | Dissolving in the first solvent and then adding to the second |
| | | | solvent |
| Ethanol | Water | 3.0/7.0 | Dissolving in the first solvent and then adding to the second solvent |
| n-Propanol | Water | 2.0/10.0 | Dissolving in the first solvent and then adding to the second solvent |
| Tetrahydrofuran | Water | 4.0/16.0 | Dissolving in the first solvent and then adding to the second solvent |

(continued)

| The first solvent | The second solvent | Volume ratio of the first solvent to the second solvent | Addition manner |
|---|---|---|---|
| Dimethyl sulfoxide | Water | 3.0/2.0 | Dissolving in the first solvent and then adding to the second solvent |
| Acetone | Water | 9.0/11.0 | Dissolving in the first solvent and then adding to the second solvent |
| Methanol | Isopropyl ether | 2.0/18.0 | Dissolving in the first solvent and then adding to the second solvent |
| Acetone | Methyl tert-butyl ether | 9.0/11.0 | Dissolving in the first solvent and then adding the second solvent to the first solvent |
| 1,4-Dioxane | Isopropyl ether | 4.0/16.0 | Dissolving in the first solvent and then adding to the second solvent |
| Chloroform | Isopropyl ether | 1.0/19.0 | Dissolving in the first solvent and then adding to the second solvent |
| Tetrahydrofuran | n-Heptane | 2.2/7.0 | Dissolving in the first solvent and then adding to the second solvent |
| Butanone | Methylcyclohexane | 7.2/13.0 | Dissolving in the first solvent and then adding to the second solvent |
| Ethyl acetate | n-Heptane | 12.0/8.0 | Dissolving in the first solvent and then adding to the second solvent |

In one or more embodiments, in the cooling crystallization method, dissolving the compound of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide in the first solvent or the mixed solvent of the first solvent and the second solvent at the starting temperature, cooling to room temperature or 4 °C, centrifugating after precipitation of solids, and drying the solid under the condition of a temperature of 20 °C to 30 °C (such as 20 °C, 21 °C, 22 °C, 23 °C, 24 °C, 25 °C, 26 °C, 27 °C, 28 °C, 29 °C, or 30 °C) and a pressure of -0.08 to -0.1MPa (such as -0.08 MPa, -0.09 MPa or -0.1 MPa) to obtain a crystalline substance.

Preferably, the starting temperature, the cooling mode, and the solvents are as described in the following table:

| Starting temperature | Cooling mode | The first solvent | The second solvent | Volume ratio of the first solvent to the second solvent |
|---|---|---|---|---|
| 60°C | Cooling to room temperature | Ethanol | - | - |
| 60°C | | Acetone | - | - |
| 60°C | | Ethyl acetate | - | - |
| 60°C | | Methanol | Water | 1.0/0.2 |
| 60°C | | Ethanol | Water | 1.0/0.2 |
| 60°C | | Isopropyl alcohol | Water | 1.0/0.2 |
| 60°C | | Tetrahydrofuran | Water | 1.0/0.2 |
| 60°C | | Dimethyl sulfoxide | Water | 1.6/0.1 |
| 60°C | | Acetone | Water | 1.2/0.2 |
| 60°C | | Ethanol | Isopropyl ether | 1.0/0.2 |
| 60°C | | Butanone | Methyl tert-butyl ether | 1.0/0.2 |
| 60°C | | Isopropyl acetate | Methyl tert-butyl ether | 5.0/1.0 |
| 60°C | | Tetrahydrofuran | Methyl tert-butyl ether | 1.0/0.2 |
| 60°C | | Isopropyl alcohol | Acetonitrile | 1.0/0.2 |
| 60°C | | Sec-butanol | n-Heptane | 0.4/0.1 |
| 60°C | | Acetone | n-Heptane | 1.0/0.2 |
| 60°C | Cooling to 4°C | 1,4-Dioxane | - | - |
| 60°C | | 1,4-Dioxane | Methyl tert-butyl ether | 1.0/0.2 |

The structure of the compound (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide is as follows:

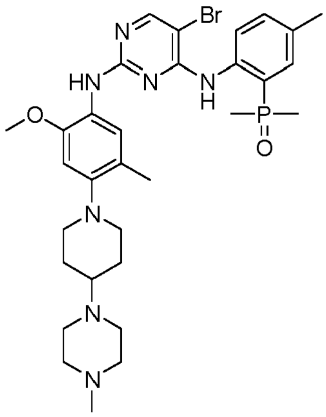,

which is an excellent EGFR and/or ALK inhibitors.

The structure of the compound (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide dihydrate is as follows:

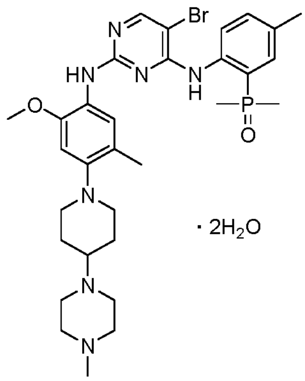

,

which has good stability and solubility, and is not hygroscopic, and can achieve a balance between various properties to meet the requirements of pharmaceutical crystal form.

BRIEF DESCRIPTION OF THE DRAWINGS

Figure 1 shows the XRPD pattern of crystal form 1.
Figure 2 shows the XRPD pattern of crystal form 2.
Figure 3 shows the XRPD pattern of crystal form 3.
Figure 4 shows the purity test pattern of crystal form 1.
Figure 5a shows the TGA pattern of crystal form 1.
Figure 5b shows the TGA pattern of crystal form 1.
Figure 6 shows the DSC pattern of crystal form 1.
Figure 7 shows the FT-IR pattern of crystal form 1.
Figure 8 shows the variable temperature XRPD pattern of crystal form 1.
Figure 9 shows the XRPD pattern of crystal form 1 for 6 months under the accelerated test condition.

DETAILED DESCRIPTION

Example 1-1: Preparation of (2-((5-bromo-2-((2-methoxy-5-methyl-4-(4-(4-methylpiperazin-1-yl)piperidin-1-yl)phenyl) amino) pyrimidin-4-yl)amino)-5-methylphenyl)dimethylphosphorus oxide

Step 1: Preparation of DA01

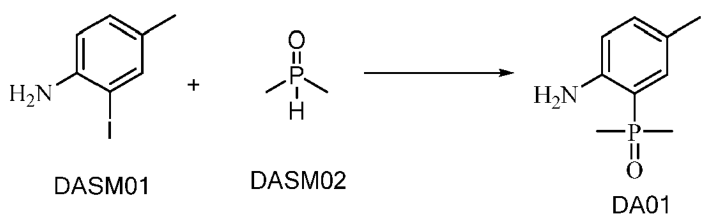

DASM01 DASM02 DA01

2-iodo-4-methylaniline (DASM01, 96.0 g, 0.412 mol, 1.0 eq), $K_2CO_3$ (79.68 g, 0.577 mol, 1.4 eq), Xantphos (6.24 g, 0.0165 mol, 0.04 eq) and DMF 576 mL were added to a 1.0 L of three-mouth bottle, the air therein was replaced with nitrogen for 2 times, then $Pd(OAc)_2$ (3.7 g, 0.0165 mol, 0.04 eq) and methylphosphinoylmethane (DASM02, 38.58 g, 0.494 mol, 1.2 eq) were added thereto under the nitrogen bubbling, and after the addition the nitrogen bubbling was continued for 15 min. After the bubbling was completed, the temperature was raised to 100 °C under the nitrogen atmosphere until the reaction was completed by TLC detection (about 2-3 h). After the reaction was completed, the reaction solution was cooled to room temperature, 1.72 L of water was added dropwise, stirred at room temperature for 30min, then filtered, the filtrate was collected, 86.0 g of sodium chloride was added to the filtrate and stirred to be dissolved, and then extracted with 1.0 L of dichloromethane for 2 times respectively, the aqueous phase was extracted with 1.0 L of mixed solvent of DCM/MeOH (10:1) for 1 time, and the organic phase was combined, dried with anhydrous sodium sulfate, concentrated under reduced

pressure to obtain an oily substance, added 48mL of ethyl acetate to the oily substance and stirred evenly, then added 144 mL of petroleum ether or n-heptane to slurry for 3 h, filtered, the filter cake was washed with 100 mL of mixed solvent of petroleum ether/ethyl acetate (1:1), and then the solid filter cake was air dried at 50 °C to obtain a total of 66.80 g of DA01 product, the yield was 88.5%, $^1$H NMR (400MHz, DMSO-d6) δ= 9.21(brs, 2H), 7.57(d, J=13.6, 1H), 7.43(d, J=8.4, 1H), 7.26-7.23(m, 1H), 2.33(s, 3H), 1.85(s, 3H), 1.82(s, 3H).

Step 2: Preparation of DA02

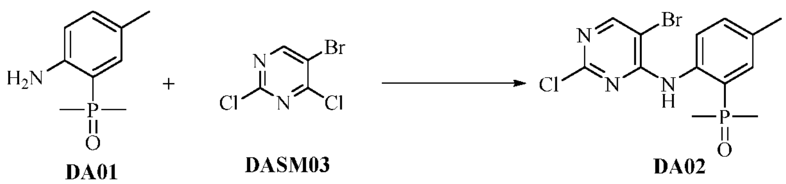

DA01 DASM03 DA02

DA01 (50.0 g, 0.273 mol, 1.0 eq), $K_2CO_3$ (45.33 g, 0.328 mol, 1.2 eq) and DMSO 300 mL were added to a 500 mL of three-mouth bottle, stirred evenly, added with 5-bromo-2,4-dichloropyrimidine (DASM03, 68.36 g, 0.30 mol, 1.1 eq), and then reacted at 60 °C until the reaction was completed by TLC detection (about 3 h). After the reaction was completed, the reaction solution was cooled to room temperature, 900 mL of water was added dropwise, a large number of solid products were precipitated, stirred for 2 h at room temperature, filtered, the filter cake was washed and stirred with 200 mL of water for 2 times, and the filter cake was collected and air dried at 60 °C to obtain a total of 94.0 g of DA02 product, the yield was 92.00%, $^1$H NMR (400 MHz, CDCl3) δ = 11.19 (s, 1H), 8.43 (dd, J = 8.4, 4.8 Hz, 1H), 8.32 (s, 1H), 7.41 (d, J = 8.8 Hz, 1H), 7.08 (m, 1H), 2.38 (s, 3H), 1.86 (s, 3H), 1.83 (s, 3H). MS-ESI (m/z): 395.9642 $(M+Na)^+$.

Step 3: Preparation of compound DA

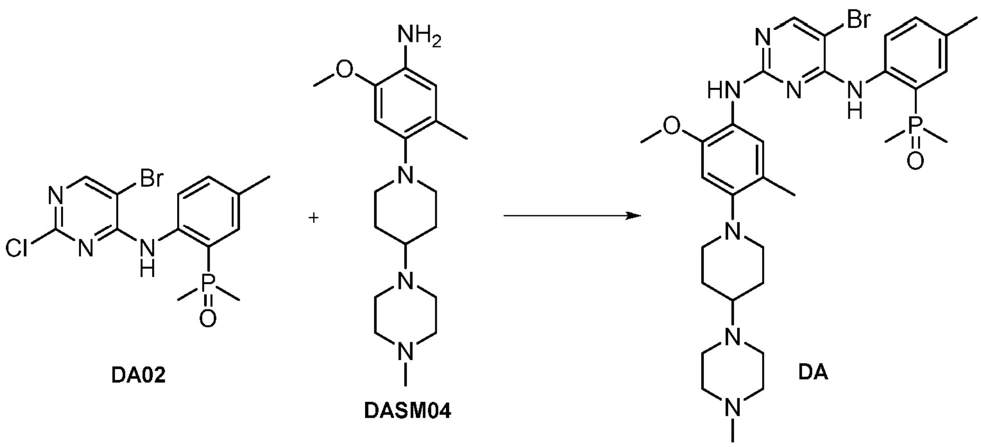

DA02 DASM04 DA

Compound DA02 (32.08 g, 85.64 mmol), compound DASM04 (30 g, 94.20 mmol, 1.1 eq), trifluoroacetic acid (24.41 g, 214.09 mmol, 2.5 eq), and 256 mL of ethylene glycol monomethyl ether were added to the reaction flask, and then heated to 100 °C to react until the reaction was completed by TLC detection (about 6 h). After the reaction was completed, the reaction solution was cooled to room temperature, 256 mL of saturated sodium bicarbonate aqueous solution and 512 mL of water were added, stirred at room temperature for 1 h, then filtered, washed with water, the resulting filter cake was stirred at room temperature for about 1 h with a mixed solvent of 150 mL DCM/EA=2/1, then filtered, the filter cake was refluxed and dissolved with 230 mL of methanol, and then stirred at room temperature for about 4 h, filtered, the filter cake was washed with a small amount of methanol, and then the filter cake was vacuum dried at 40 °C to 50 °C to obtain a dry product of compound DA (i.e., (2-((5-Bromo-2-((2-methoxy-5-methyl-4-(4-(4-methylpiperazin-1-yl)piperidin-1-yl)phenyl) amino) pyrimidin-4-yl)amino)-5-methylphenyl)dimethylphosphorus oxide, 37.0 g, yield 65.8%). $^1$H NMR (400 MHz, CDCl3) δ = 10.29 (s, 1H), 8.32 (dd, J = 8.8, 4.8 Hz, 1H), 8.18 (s, 1H), 8.01 (s, 1H), 7.44-7.30 (m, 2H), 7.11 (d, J = 14.0Hz, 1H), 6.61 (s, 1H), 3.85 (s, 3H), 3.15 (d, J = 11.6 Hz, 2H), 2. 80-2.52 (m, 11H), 2.38 (s, 3H), 2.35 (s, 3H), 2.14 (s, 3H), 1.96 (d, J = 11.0 Hz, 2H), 1.85 (s, 3H), 1.82 (s, 3H), 1.78-1.68 (m, 2H). MS-ESI (m/z) : 656.2459 $(M+H)^+$.

Examples 1-2: Preparation of crystal form of compound (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide dihydrate

Ethyl acetate (22.19 kg) and water (2.72 kg) (9:1) were added to the 30 L reactor, followed by compound DA (i.e., (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide, 1.88 kg), slurried at 10 °C to 30 °C for 50 min to 70 min, then filtered by suction, the filter cake was washed with 3.07 kg of ethyl acetate, and after washing the wet filter cake was transferred to a vacuum drying oven, dried to 5% to 6% moisture at a temperature of 20 °C to 30 °C and a pressure of -0.08 MPa to -0.1 MPa, and the resulting substance was collected to obtain a crystalline compound, i.e., 1.84 kg of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide dihydrate with purity of 99.66%, single impurity ≤0.06% (see Figure 4 for purity detection pattern), melting point: 213 °C to 217 °C.

Example 2: XRPD determination of crystal form of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide dihydrate

The determination was carried out according to The second method - Powder X-ray diffraction method of X-ray diffraction in General Principles 0451 of Part IV of the Chinese Pharmacopoeia (Edition 2020):

Table 1. Powder XRPD test parameters

| | |
|---|---|
| Device | X-ray Diffractometer (XRPD) |
| Model | Bruker D8 Advance Diffractometer |
| Scanning Type | Locked Coupled |
| Scanning Method | Continuous Scanning |
| Detector | SSD160-2 |
| X-ray Source | Cu-$K_\alpha$ |
| Divergent Slit | 0.6 mm |
| Voltage/Current | 40 KV, 40 mA |
| Step Size | 0.02° 2θ |
| Scan Speed | 0.2 sec/step |
| Starting Angle | 3° |

The XRPD pattern of the crystal form of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide dihydrate is shown in Figure 1, the XRPD data are shown in Table 2, and the crystal form is named crystal form 1.

Table 2. XRPD data of crystal form 1

| **No.** | 2θ | Intensity | **No.** | 2θ | Intensity | **No.** | 2θ | Intensity | **No.** | 2θ | Intensity |
|---|---|---|---|---|---|---|---|---|---|---|---|
| **1** | 5.54° | 100.00% | **12** | 19.47° | 5.90% | **23** | 26.27° | 13.90% | **34** | 33.64° | 10.40% |
| **2** | 8.50° | 5.40% | **13** | 19.78° | 3.20% | **24** | 26.48° | 12.50% | **35** | 34.17° | 5.40% |
| **3** | 10.53° | 2.50% | **14** | 20.05° | 1.90% | **25** | 26.93° | 4.90% | **36** | 35.18° | 1.80% |
| **4** | 11.06° | 39.50% | **15** | 21.18° | 15.10% | **26** | 27.35° | 6.70% | **37** | 37.30° | 1.30% |
| **5** | 11.48° | 3.70% | **16** | 21.76° | 1.30% | **27** | 27.90° | 23.80% | **38** | 37.77° | 1.20% |
| **6** | 13.00° | 2.40% | **17** | 22.22° | 4.40% | **28** | 29.11° | 2.40% | **39** | 38.26° | 3.30% |
| **7** | 16.15° | 13.10% | **18** | 22.66° | 3.50% | **29** | 29.35° | 6.60% | **40** | 40.90° | 1.00% |
| **8** | 16.73° | 14.40% | **19** | 23.11° | 5.60% | **30** | 29.70° | 1.60% | **41** | 43.08° | 3.30% |
| **9** | 17.10° | 16.30% | **20** | 23.50° | 3.70% | **31** | 31.33° | 1.30% | **42** | 43.37° | 5.70% |
| **10** | 18.34° | 2.00% | **21** | 24.16° | 14.20% | **32** | 31.77° | 2.20% | **43** | 45.39° | 4.80% |

(continued)

| **No.** | 2θ | Intensity | **No.** | 2θ | Intensity | **No.** | 2θ | Intensity | **No.** | 2θ | Intensity |
|---|---|---|---|---|---|---|---|---|---|---|---|
| **11** | 19.12° | 53.10% | **22** | 25.66° | 2.10% | **33** | 32.22° | 4.10% | | | |

Example 3: TGA determination of the crystal form 1

Table 3. TGA Test Parameters:

| Thermogravimetric Analyzer (TGA) | | |
|---|---|---|
| Instrument | Model | TGA2 |
| | Control software | STARe |
| | Analysis software | STARe |
| | Sample tray | Alumina crucible |
| Parameters | Sample weight | 1 mg |
| | Protective gas | Nitrogen |
| | Gas flow rate | 50 mL/min |
| | Detection method | 35.0 °C to 105.00 °C<br>Heating rate: 20.00 °C /min under 105.00 °C for 50 min |

The TGA detection patterns of the crystal form 1 are shown in Figures 5a and 5b.

The test results show that there is a significant water loss step (weight loss step) between about 50 °C and 80 °C for crystal form 1, and the weight loss is about 5.7%.

Example 4: DSC determination of the crystal form 1

Table 4. DSC Test Parameters:

| Differential Scanning Calorimetry (DSC) | | |
|---|---|---|
| Instrument | Model | TA Instruments Q200 DSC |
| | Control software | Thermal Advantage |
| | Analysis software | Universal Analysis |
| | Sample tray | Aluminum crucible (capped and perforated) |
| Parameters | Tested sample | 0.5 mg-5 mg |
| | Protective gas | Nitrogen |
| | Gas flow rate | 50 mL/min |

The DSC detection pattern of the crystal form 1 is shown in Figure 6. The DSC pattern shows that there is an obvious endothermic peak for crystal form 1 between about 50 °C and 80 °C.

Example 5. Fourier transform infrared spectroscopy (FT-IR) determination of the crystal form 1

Table 5. FT-IR Test Parameters:

| Fourier transform infrared spectroscopy (FT-IR) | | |
|---|---|---|
| Instrument | Model | Bruker Tensor27 |
| | No. | LY-01-001 |
| | Control software | OPUS |
| | Analysis software | Omnic |
| Parameters | Detection method | ATR method |
| | Acquisition wavelengths range | 600 $cm^{-1}$ to 4000 $cm^{-1}$ |
| | Scanning time | 32 s |
| | Resolution | 4 $cm^{-1}$ |

The FT-IR detection pattern of the crystal form 1 is shown in Figure 7.

Example 6: Preparation of crystal form 2 of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide

30 mg of compound DA was taken, added with 1.0 mL of dichloromethane to dissolve, and then concentrated to dry under reduced pressure on a rotary evaporator at 30°C to obtain crystalline substance. X-ray powder diffraction (XRPD) test was performed immediately afterwards. The XRPD pattern is shown in Figure 2, and the XRPD data is shown in Table 6, named crystal form 2.

Table 6. XRPD data of the crystal form 2

| No. | 2θ | Intensity | No. | 2θ | Intensity | No. | 2θ | Intensity |
|---|---|---|---|---|---|---|---|---|
| **1** | 5.72 | 100% | **11** | 18.71 | 9.5% | **21** | 26.36 | 6.9% |
| **2** | 10.24 | 35.4% | **12** | 19.49 | 27.4% | **22** | 27.22 | 6% |
| **3** | 11.44 | 36.2% | **13** | 20.07 | 23.5% | **23** | 27.51 | 6.6% |
| **4** | 15.07 | 5.1% | **14** | 21.59 | 15.5% | **24** | 28.45 | 7% |
| **5** | 15.09 | 4.8% | **15** | 22.41 | 15.8% | **25** | 28.75 | 5.8% |
| **6** | 15.92 | 16.1% | **16** | 23.05 | 7.2% | **26** | 29.49 | 6.4% |
| **7** | 16.41 | 57.5% | **17** | 23.25 | 13.7% | **27** | 29.95 | 7.4% |
| **8** | 17.07 | 15.7% | **18** | 23.55 | 10.9% | **28** | 31.93 | 5.2% |
| **9** | 17.38 | 26.1% | **19** | 24.71 | 7.2% | **29** | 36.71 | 4% |
| **10** | 18.49 | 16.7% | **20** | 25.19 | 18.1% | | | |

Example 7: Preparation of crystal form 3 of (2-((5-bromo-2-((2-methoxy-5-methyl-4-(4-(4-methylpiperazin-1-yl)piperidin-1-yl)phenyl)amino) pyrimidin-4-yl)amino)-5-methylphenyl)dimethylphosphorus oxide

30 mg of compound DA was taken, added with 1.0 mL of trifluoroethanol to dissolve, and then concentrated to dry under reduced pressure on a rotary evaporator at 30°C to obtain crystalline substances. X-ray powder diffraction (XRPD) test was performed immediately afterwards. The XRPD pattern is shown in Figure 3, and the XRPD data is shown in Table 7, named crystal form 3.

Table 7. XRPD data of the crystal form 3

| No. | 2θ | Intensity | No. | 2θ | Intensity | No. | 2θ | Intensity |
|---|---|---|---|---|---|---|---|---|
| **1** | 4.86 | 17.4 | **10** | 17.21 | 100 | **19** | 28.07 | 10.3 |
| **2** | 5.62 | 11.1 | **11** | 18.3 | 31.5 | **20** | 28.77 | 16.7 |
| **3** | 6.88 | 12.2 | **12** | 19.73 | 59.9 | **21** | 29.09 | 11.1 |

(continued)

| No. | 2θ | Intensity | No. | 2θ | Intensity | No. | 2θ | Intensity |
|---|---|---|---|---|---|---|---|---|
| **4** | 8.16 | 11.6 | **13** | 20.67 | 34.7 | **22** | 30.39 | 15.6 |
| **5** | 10.54 | 21.9 | **14** | 21.43 | 59.6 | **23** | 30.79 | 7.6 |
| **6** | 12.31 | 13.6 | **15** | 22.97 | 38.3 | **24** | 32.95 | 9.3 |
| **7** | 14.32 | 8.3 | **16** | 23.69 | 26.9 | **25** | 34.55 | 10 |
| **8** | 15.92 | 32.1 | **17** | 25.51 | 20.4 | **26** | 35.32 | 10.3 |
| **9** | 16.22 | 24.1 | **18** | 27.59 | 10.2 | | | |

Example 8: Determination of moisture of the crystal form 1 (Fischer method)

The determination was carried out according to the first method 1 of General Principles 0832 of Part IV of the Chinese Pharmacopoeia (Edition 2020 ):

Experimental instruments and equipment

Electronic balance: Model Sartorius BSA 224S
Water titrator: Model Metrohm 888 Titrando
Experimental materials
Karl Fischer reagent: Shanghai McLean Biochemical Technology Co., Ltd., Batch No.: C12156591
Solvent: methanol anhydrous, purchased from Tedia, Batch No.: 710901
Test product information
Crystal form 1: prepared in Examples 1-2

The test results are shown in Table 8:

Table 8

| Karl Fischer reagent titer | | | | |
|---|---|---|---|---|
| Water weight | Consumption of titrant (mL) | Titer (mg/mL) | Mean titer (mg/mL) | RSD (%) |
| 0.0150 | 3.8695 | 3.8765 | 3.8713 | 0.15 |
| 0.0148 | 3.8290 | 3.8652 | | |
| 0.0149 | 3.8480 | 3.8721 | | |
| Moisture content determination of the test sample of the crystal form 1 | | | | |
| Test sample | Weight of test sample (g) | Consumption of titrant (mL) | Moisture content (%) | |
| Crystal form 1 | 0.2590 | 3.6155 | 5.40 | |

The moisture content was measured to be 5.40% according to the first method 1 of General Principles 0832 of Part IV of the Chinese Pharmacopoeia (Edition 2020 ), which corresponded to the TGA water loss in Example 3, and both of them confirmed that crystal form 1 contained 2 molecules of water.

Example 9: Determination of moisture of the crystal form 2 and the crystal form 3 (Fischer method)

The determination was carried out according to the first method 1 of General Principles 0832 of Part IV of the Chinese Pharmacopoeia (Edition 2020 ).

Experimental instruments and equipment
Electronic balance: Model Sartorius BSA 224S
Water titrator: Model Metrohm 888 Titrando

Experimental materials
Karl Fischer reagent: Shanghai McLean Biochemical Technology Co., Ltd., Batch No.: C12156591
Solvent: methanol anhydrous, purchased from Tedia, Batch No.: 710901
Test product information
Crystal form 2: prepared in Example 6
Crystal form 3: prepared in Example 7

The test results are shown in Table 9:

Table 9

| Karl Fischer reagent titer | | | | |
|---|---|---|---|---|
| Water weight | Consumption of titrant (mL) | Titer (mg/mL) | Mean titer (mg/mL) | RSD (%) |
| 0.0150 | 3.4095 | 4.3995 | 4.4015 | 0.47% |
| 0.0151 | 3.4140 | 4.4230 | | |
| 0.0148 | 3.3775 | 4.3819 | | |
| Determination of the moisture content of the test samples | | | | |
| Test Sample | Weight of Test Sample (g) | Consumption of Titrant (mL) | Moisture Content (%) | |
| Crystal form 2 | 0.1680 | 0.4520 | 1.18 | |
| Crystal form 3 | 0.2131 | 0.2460 | 0.51 | |

The moisture contents were measured to be 1.18% and 0.51% respectively of the crystal form 2 and the crystal form 3 according to the first method 1 of General Principles 0832 of Part IV of the Chinese Pharmacopoeia (Edition 2020 ).

Example 10: Variable temperature XRPD of the crystal form 1

The crystalline form 1 was tested on a hot-stage XRPD detection instrument to test the XRPD patterns under different temperature conditions, as shown in Figure 8.

When the temperature on the hot-stage XRPD detection instrument was 50°C, the crystal form of crystal form 1 remained unchanged, when the temperature reached to 80°C and 120°C, the crystal form 1 lost two moles of crystal water, and became an unstable free crystal form that can only be monitored on the hot-stage XRPD detection instrument, and once the temperature was cooled down to room temperature, the unstable free crystal form was transformed into crystal form 1 again, which proved that the stability of the free crystal form was poor, and it was easy to transform into crystal form 1 at room temperature.

Example 11: Other preparation methods for the crystal form 1

(1) Volatile crystallization method

Experimental operation: compound DA sample was added to solvent 1 or a mixed solvent of solvent 1 and solvent 2 at the temperature shown in Table 10, the solid sample was ultrasonically dissolved, and then filtered, the filtrate was exposed at the corresponding temperature to volatilize dry, and the solid was taken for characterization. The characterization results are shown in Table 10.

Table. 10

| **No.** | **Temperature (°C)** | **Solvent 1** | **Solvent 2** | **Solvent 1/ Solvent 2 (mL)** | **Analysis of result** |
|---|---|---|---|---|---|
| 1 | 40 | Methanol | - | 2.0 | Crystal form 1 |
| 2 | Room temperature | Ethanol | - | 3.0 | Crystal form 1 |
| 3 | 40 | Isopropanol | - | 3.0 | Crystal form 1 |
| 4 | Room | Acetone | - | 7.0 | Crystal form |
| | temperature | | | | 1 |

(continued)

| No. | Temperature (°C) | Solvent 1 | Solvent 2 | Solvent 1/ Solvent 2 (mL) | Analysis of result |
|---|---|---|---|---|---|
| 5 | 40 | Ethyl acetate | - | 20.0 | Crystal form 1 |
| 6 | Room temperature | Tetrahydrofuran | - | 5.0 | Crystal form 1 |
| 7 | Room temperature | Chloroform | - | 1.0 | Crystal form 1 |
| 8 | Room temperature | Methanol | Water | 2.0/0.1 | Crystal form 1 |
| 9 | Room temperature | Ethanol | Water | 3.0/0.1 | Crystal form 1 |
| 10 | Room temperature | Acetone | Water | 5.0/0.1 | Crystal form 1 |
| 11 | Room temperature | Tetrahydrofuran | Water | 5.0/0.1 | Crystal form 1 |
| 12 | 40 | Methanol | Ethyl acetate | 2.0/1.0 | Crystal form 1 |
| 13 | Room temperature | Ethanol | n-Heptane | 3.0/1.0 | Crystal form 1 |
| 14 | Room temperature | Acetone | Methyl tert-butyl ether | 9.0/3.0 | Crystal form 1 |
| 15 | Room temperature | Butanone | Acetonitrile | 8.0/3.0 | Crystal form 1 |
| 16 | Room temperature | Tetrahydrofuran | n-Heptane | 5.0/2.0 | Crystal form 1 |
| 17 | 40 | Chloroform | Methylcyclohexane | 1.0/1.0 | Crystal form 1 |
| Note: Room temperature herein refers to 10°C to 30°C, such as 20°C, and 25°C. | | | | | |

(2) Slurry crystallization method

Experimental operation: about 30 mg of compound DA sample was added to the corresponding solvent 1 or a mixed solvent of solvent 1/solvent 2 to obtain a suspension at the temperature shown in Table 11, after slurrying the suspension for 3 days , centrifuged, the solid was dried overnight under the conditions of temperature of 20 °C to 30 °C and pressure of -0.08 MPa to -0.1MPa, and then the solid was taken for XRPD characterization. The characterization results are shown in Table 11.

Table 11

| No. | Temperature (°C) | Solvent 1 | Solvent 2 | Solvent 1/ Solvent 2 (mL) | Analysis of result |
|---|---|---|---|---|---|
| 1 | 4 | Methanol | - | 2.0 | Crystal form 1 |
| 2 | Room temperature | Ethanol | - | 1.0 | Crystal form 1 |
| 3 | Room temperature | Water | - | 2.0 | Crystal form 1 |
| 4 | 4 | Acetone | - | 1.0 | Crystal form 1 |
| 5 | 40 | Isopropyl ether | - | 2.0 | Crystal form |
| | | | | | 1 |
| 6 | Room temperature | Ethyl acetate | - | 2.0 | Crystal form 1 |
| 7 | Room temperature | Methyl tert-butyl ether | - | 2.0 | Crystal form 1 |
| 8 | 40 | Isopropyl acetate | - | 2.0 | Crystal form 1 |
| 9 | 4 | Tetrahydrofuran | - | 1.0 | Crystal form 1 |
| 10 | Room temperature | Acetonitrile | - | 2.0 | Crystal form 1 |
| 11 | Room temperature | Toluene | - | 2.0 | Crystal form 1 |
| 12 | Room temperature | n-Heptane | - | 1.0 | Crystal form 1 |
| 13 | Room temperature | Methanol | Water | 1.0/1.0 | Crystal form 1 |
| 14 | Room temperature | Ethanol | Water | 1.0/1.0 | Crystal form 1 |

(continued)

| No. | Temperature (°C) | Solvent 1 | Solvent 2 | Solvent 1/ Solvent 2 (mL) | Analysis of result |
|---|---|---|---|---|---|
| 15 | Room temperature | Acetone | Water | 1.5/0.5 | Crystal form 1 |
| 16 | Room temperature | Water saturated with ethyl acetate | | 2.0 | Crystal form 1 |
| 17 | 4 | Tetrahydrofuran | Water | 0.8/1.2 | Crystal form 1 |
| 18 | Room temperature | 1,4-Dioxane | Water | 0.7/1.3 | Crystal form 1 |
| 19 | 40 | Acetonitrile | Water | 1.4/0.6 | Crystal form 1 |
| 20 | Room temperature | Dimethyl sulfoxide | Water | 1.0/1.0 | Crystal form 1 |
| 21 | 4 | Ethanol | n-Heptane | 1.0/1.0 | Crystal form 1 |
| 22 | Room temperature | Isopropanol | Isopropyl ether | 1.0/1.0 | Crystal form 1 |
| 23 | Room temperature | Acetone | Methyl tert-butyl ether | 1.0/1.0 | Crystal form 1 |
| 24 | 40 | Butanone | Toluene | 1.0/1.0 | Crystal form 1 |
| 25 | Room temperature | Ethyl acetate | Methyl tert-butyl ether | 1.0/1.0 | Crystal form 1 |
| 26 | 40 | Isopropyl acetate | Methylcyclohexane | 1.0/1.0 | Crystal form 1 |
| 27 | Room temperature | Tetrahydrofuran | n-Heptane | 0.5/1.5 | Crystal form 1 |
| 28 | Room temperature | 1,4-Dioxane | Isopropyl ether | 0.5/1.5 | Crystal form 1 |
| 29 | Room temperature | Acetonitrile | Butyl acetate | 1.0/1.0 | Crystal form 1 |
| 30 | 40 | Acetonitrile | Methyl tert-butyl | 0.8/1.2 | Crystal form |
| | | | ether | | 1 |
| 31 | Room temperature | Chloroform | Methylcyclohexane | 0.5/1.5 | Crystal form 1 |
| Note: Room temperature herein refers to 10°C to 30°C, such as 20°C, and 25°C. | | | | | |

(3) Antisolvent crystallization method

Experimental operation: about 20 mg of compound DA sample was added to solvent 1 to obtain a settled solution. Solvent 2 was slowly added to the settled solution of solvent 1 under stirring (forward addition), or the settled solution of solvent 1 was added to solvent 2 (reverse addition), the solid was precipitated and stirred overnight, centrifuged, vacuum dried overnight at room temperature, and the solid was taken for XRPD characterization; if there was no crystal precipitation, the solution was transferred to 4°C and stirred overnight, and after the solid was precipitated, centrifuged. Under the conditions of temperature of 20°C to 30°C and pressure of -0.08 MPa to -0.1MPa, the solids were dried overnight and then taken for XRPD characterization. The characterization results are shown in Table 12.

Table 12

| No. | Solvent 1 | Solvent 2 | Solvent 1/ Solvent 2 (mL) | Method | Analysis of result |
|---|---|---|---|---|---|
| 1 | Methanol | Water | 2.0/3.0 | Forward | Crystal form 1 |
| 2 | Ethanol | Water | 3.0/7.0 | Forward | Crystal form 1 |
| 3 | n-Propanol | Water | 2.0/10.0 | Forward | Crystal form 1 |
| 4 | Tetrahydrofuran | Water | 4.0/16.0 | Forward | Crystal form 1 |
| 5 | Dimethyl sulfoxide | Water | 3.0/2.0 | Forward | Crystal form 1 |
| 6 | Acetone | Water | 9.0/11.0 | Forward | Crystal form 1 |
| 7 | Methanol | Isopropyl ether | 2.0/18.0 | Forward | Crystal form 1 |
| 8 | Acetone | Methyl tert-butyl ether | 9.0/11.0 | Reverse | Crystal form 1 |
| 9 | 1,4-Dioxane | Isopropyl ether | 4.0/16.0 | Forward | Crystal form 1 |

(continued)

| No. | Solvent 1 | Solvent 2 | Solvent 1/ Solvent 2 (mL) | Method | Analysis of result |
|---|---|---|---|---|---|
| 10 | Chloroform | Isopropyl ether | 1.0/19.0 | Forward | Crystal form 1 |
| 11 | Tetrahydrofuran | n-Heptane | 2.2/7.0 | Forward | Crystal form 1 |
| 12 | Butanone | Methylcyclohexane | 7.2/13.0 | Forward | Crystal form 1 |
| 13 | Ethyl acetate | n-Heptane | 12.0/8.0 | Forward | Crystal form 1 |
| Note: Room temperature herein refers to 10°C to 30°C, such as 20°C, and 25°C. | | | | | |

(4) Cooling crystallization method

Experimental operation: about 20 mg of compound DA sample was added to the corresponding solvent 1 or a mixed solvent of solvent 1/solvent 2 to form a solution at the start temperature (60 °C), then stirred at the end temperature, after the solid was precipitated, stirred overnight, centrifuged, the solid was dried overnight under the conditions of temperature of 20 °C to 30 °C and pressure of -0.08 MPa to -0.1MPa, and the solid was taken for XRPD characterization. The results are shown in Table 13.

Table 13

| No. | Start and end temperatures (°C) | Cooling method | Solvent 1 | Solvent 2 | Solvent 1/ Solvent 2 (mL) | Analysis of result |
|---|---|---|---|---|---|---|
| 1 | 60-RT | Naturally cooled to room temperature | Ethanol | - | 1.0 | Crystal form 1 |
| 2 | 60-RT | | Acetone | - | 2.0 | Crystal form 1 |
| 3 | 60-RT | | Ethyl acetate | - | 2.0 | Crystal form 1 |
| 4 | 60-RT | | Methanol | Water | 1.0/0.2 | Crystal form 1 |
| 5 | 60-RT | | Ethanol | Water | 1.0/0.2 | Crystal form 1 |
| 6 | 60-RT | | Isopropanol | Water | 1.0/0.2 | Crystal form 1 |
| 7 | 60-RT | | Tetrahydrofuran | Water | 1.0/0.2 | Crystal form 1 |
| 8 | 60-RT | | Dimethyl sulfoxide | Water | 1.6/0.1 | Crystal form 1 |
| 9 | 60-RT | | Acetone | Water | 1.2/0.2 | Crystal form 1 |
| 10 | 60-RT | | Ethanol | Isopropyl ether | 1.0/0.2 | Crystal form 1 |
| 11 | 60-RT | | Butanone | Methyl tert-butyl ether | 1.0/0.2 | Crystal form 1 |
| 12 | 60-RT | | Isopropyl acetate | Methyl tert-butyl ether | 5.0/1.0 | Crystal form 1 |
| 13 | 60-RT | | Tetrahydrofuran | Methyl tert-butyl ether | 1.0/0.2 | Crystal form 1 |
| 14 | 60-RT | | Isopropanol | Acetonitrile | 1.0/0.2 | Crystal form 1 |
| 15 | 60-RT | | Sec-butanol | n-Heptane | 0.4/0.1 | Crystal form 1 |
| 16 | 60-RT | | Acetone | n-Heptane | 1.0/0.2 | Crystal form 1 |
| 17 | 60-4 | Naturally cooled to room temperature, | 1,4-Dioxane | - | 1.0 | Crystal form 1 |
| 18 | 60-4 | | 1,4-Dioxane | Methyl tert-butyl | 1.0/0.2 | Crystal form 1 |
| | | then stirred overnight at 4°C | | ether | | |

Example 12: Comparative experiment of hygroscopicity

Hygroscopicity test was carried out on the compound DA, crystal form 1 (prepared in Examples 1-2), crystal form 2 and crystal form 3 obtained in the above Examples according to the "Guiding Principles for the Hygroscopicity of Drugs" of Part IV of the Chinese Pharmacopoeia (Edition 2020 ).

The description of hygroscopic characteristics and the definition of hygroscopic weight gain are as follows:

Deliquescence: absorbs sufficient amount of water to form a liquid;
Very hygroscopic: the weight gain by hygroscopy is not less than 15%;
Hygroscopic: the weight gain by hygroscopy is less than 15% but not less than 2%;
Slightly hygroscopic: the weight gain by hygroscopy is less than 2% but not less than 0.2%; and
Not or almost not hygroscopic: the weight gain by hygroscopy is less than 0.2% from hygroscopicity.

The results are shown in Table 14:

Table 14

| Sample | Weight of weighting bottle m1/g | Sample + Weight of weighting bottle m2/g | Sample + Weight of weighting bottle after 24h m3/g | Weight gain % after 24h | Hygroscopicity |
|---|---|---|---|---|---|
| Compound DA | 17.5550 | 17.7851 | 17.7938 | 3.78% | Hygroscopic |
| Crystal form 1 | 18.1852 | 18.4284 | 18.4288 | 0.16% | Not or almost not hygroscopic |
| Crystal form 2 | 17.2974 | 17.4528 | 17.4549 | 1.35% | Slightly hygroscopic |
| Crystal form 3 | 18.2926 | 18.4468 | 18.4491 | 1.49% | Slightly hygroscopic |

Conclusion: compound DA has hygroscopic property, crystal form 2 and crystal form 3 have slight hygroscopic properties, and crystal form 1 has no or almost no hygroscopicity. Hygroscopicity will affect the mixing uniformity and content uniformity of formulation process development, and crystal form 1 can maintain better fluidity, compressibility and uniformity in the formulation process compared with crystal form 2, crystal form 3 and compound DA, which is more conducive to formulation development.

Example 13: Vacuum drying stability test

The crystal form 1 obtained in the above Examples 1-2 was taken and vacuum dried at 35 °C. It was found that the crystal form 1 had not been transformed. The moisture content of crystal form 1 was 5.26% after vacuum drying at 35°C for 24 h.

Example 14: Stability test

(1) Stability test 1 of crystal form 1 (prepared in Examples 1-2).

Experimental operation: approximately 30 mg of solid sample of crystal form 1 was taken and placed in a centrifuge tube, and left at room temperature in an open solvent atmosphere, and after a period of time (Day 4 and Day 6), the solid was taken for XRPD characterization. The results are shown in Table 15. As can be seen from Table 15, the crystal form 1 remains unchanged after standing in different solvent atmospheres for a period of time.

Table 15

| Solvent | Analysis of result | |
|---|---|---|
| | Day 4 | Day 6 |
| Ethanol | Crystal form 1 | Crystal form 1 |
| Acetone | Crystal form 1 | Crystal form 1 |
| Tetrahydrofuran | Crystal form 1 | Crystal form 1 |
| Ethyl acetate | Crystal form 1 | Crystal form 1 |

(2) Stability test 2 of crystal form 1

A sample of about 20 mg of crystal form 1 (prepared in Examples 1-2) was taken and placed in a sample vial for a period of time under different temperature and humidity conditions, and the solid was taken for XRPD characterization. The results are shown in Table 16. As can be seen from Table 16, the crystal form of crystal form 1 stayed unchanged under

different humidity conditions (low humidity and high humidity) and different temperatures (low temperature and high temperature).

Table 16

| Condition | Analysis of result of Day 4 | Analysis of result of Day 6 | Analysis of result of Day 11 |
|---|---|---|---|
| RT-12%RH | Crystal form 1 | Crystal form 1 | Crystal form 1 |
| RT-58%RH | Crystal form 1 | Crystal form 1 | Crystal form 1 |
| RT-97%RH | Crystal form 1 | Crystal form 1 | Crystal form 1 |
| 25°C-60%RH | Crystal form 1 | Crystal form 1 | Crystal form 1 |
| 40°C-75%RH | Crystal form 1 | Crystal form 1 | Crystal form 1 |

(3) Stability test 3 of crystal form 1

The stability of the crystal form 1 (prepared in Examples 1-2) under long-term and accelerated conditions was investigated, and the results showed that the crystal form of crystal form 1 remained unchanged after being placed for 10 days and 6 months under long-term and accelerated conditions, as shown in Table 17, Table 17-1, and Figure 9.

Table 17

| Crystal form | Crystal form 1 | | | | | |
|---|---|---|---|---|---|---|
| Sample dosage | 30 mg | | | | | |
| Sampling time | Day 0, Day 10, Month 6 | | | | | |
| Test item | Crystal form (XRPD) | | | | | |
| Experimental condition | Long-term (25°C±2°C, 65%RH±10%RH, exposed to air and away from light) | | | Acceleration (40°C±2°C, 75%RH±10%RH, exposed to air and away from light) | | |
| Experimental result | Day 0 | Day 10 | Month 6 | Day 0 | Day 10 | Month 6 |
| | Crystal form 1 | Crystal form 1 | Crystal form 1 | Crystal form 1 | Crystal form 1 | Crystal form 1 |

Table 17-1

| Crystal form | Crystal form 1 | | | |
|---|---|---|---|---|
| Sampling time | Day 0, Month 6 | | | |
| Test items | Traits, maximum single impurity and total impurities | | | |
| Experimental condition | Long-term (25°C±2°C, 65%RH±10%RH) | | Acceleration (40°C±2°C, 75%RH±10%RH) | |
| Experimental result | Day 0 | Month 6 | Day 0 | Month 6 |
| | Traits: off-white solid powder | Traits: off-white solid powder | Traits: off-white solid powder | Traits: off-white solid powder |

According to the Edition 2020 of the Chinese Pharmacopoeia and the guidelines for stability study of ICHQ1A, the crystal form 1 obtained from Examples 1-2 was placed under long-term (25 °C±2 °C, 65% RH±10% RH) and accelerated (40 °C±2 °C, 75% RH±10%RH) conditions for 6 months, and the changes in the traits, maximum single impurity and total impurities of crystal form 1 were detected. The results showed that the crystal form 1 was placed for 6 months under long-term and accelerated conditions, and had good stability, no change in traits, and the maximum single impurity of ≤ 0.07%, which is far less than the quality control limit of 0.15% required by the Chinese Pharmacopoeia and ICH guidelines, and the total impurities after 6 months of placement had not increased compared with that on Day 0, which meets the requirements

of pharmaceutical crystal form stability.

At the same time, crystal form 2 and crystal form 3 were placed under long-term conditions for 30 days, and the traits changed significantly, which did not meet the requirements of pharmaceutical crystal form (according to the Edition 2020 of the Chinese Pharmacopoeia and the guidelines for stability study of ICHQ1A. Crystal form 2 and crystal form 3 were placed for only 1 month under long-term (25°C±2°C, 65%RH±10%RH) conditions, and the traits changed from off-white solid powder to light yellow solid powder, which was significantly changed), as shown in Table 17-2.

Table 17-2

| Sampling time | Day 0, Day 30 | | | |
|---|---|---|---|---|
| Test item | Traits | | | |
| Experimental condition | Long-term (25°C±2°C, 65%RH±10%RH) | | Long-term (25°C±2°C, 65%RH±10%RH) | |
| Test sample | Crystal form 2 | | Crystal form 3 | |
| Experimental result | Day 0 | Day 30 | Day 0 | Day 30 |
| | Off-white solid powder | Light yellow solid powder | Off-white solid powder | Light yellow solid powder |

(4) High temperature and illumination stability test of crystal forms 1-3

The stability of crystal form 1 (prepared in Examples 1-2), crystal form 2 and crystal form 3 under illumination conditions was investigated, and the traits of crystal form 2 and crystal form 3 changed significantly under illumination conditions, all of them changed from off-white solid powder to light yellow solid powder, while the traits of crystal form 1 did not change. The test results are shown in Table 18-1.

Table 18-1

| Test sample | Crystal form 1, Crystal form 2, Crystal form 3 | | |
|---|---|---|---|
| Sampling time | Day 0, Day 10 | | |
| Test item | Traits | | |
| Experimental condition | | | Illumination (illuminance: 5000Lx) |
| Experimental result | Test sample | Day 0 | Day 10 |
| | Crystal form 1 | Traits: off-white solid powder | Traits: off-white solid powder |
| | Crystal form 2 | Traits: off-white solid powder | Traits: light yellow solid powder |
| | Crystal form 3 | Traits: off-white solid powder | Traits: light yellow solid powder |

The results show that the stability of crystal form 1 under illumination conditions is better than that of crystal form 2 and crystal form 3.

The crystal stability of crystal form 1 was investigated under high temperature and illumination conditions. The results are shown in Table 18-2, which indicates that the stability of crystal form 1 after 30 days of high temperature and illumination conditions was good, and there was no significant change in the traits, single and total impurities. The stabilities of crystal form 2 and crystal form 3 under high temperature, illumination and long-term conditions were investigated. The results are shown in Table 18-3 and Table 18-4. The experimental results show that the traits of crystal form 2 and crystal form 3 changed significantly from off-white solid powder to light yellow solid powder after 10 days or 30 days under high temperature and illumination conditions. In addition, the impurities in crystal form 2 and crystal form 3 increased very rapidly under high temperature and illumination conditions (the impurities in crystal form 2 increased from 0.63% on day 0 to 2.0% on day 30 at the fastest under illumination conditions, and the impurities in crystal form 3 increased from 0.56% on day 0 to 3.05% on day 30 at the fastest under illumination conditions), indicating that their stabilities were poorer.

Table 18-2

| Test sample | Crystal form 1 | | |
|---|---|---|---|
| Sampling time | Day 0, Day 10, Day 30 | | |
| Test item | Traits, purity, single impurity and total impurities | | |
| Experimental condition | | High temperature (50°C) | Illumination (illuminance: 5000Lx) |
| Experimental result | Day 0 | Day 10 | Day 10 |
| | Traits: off-white solid powder purity: 99.66% the number of impurities above the reporting limit is 4 total impurities: 0.34% (The reporting limit follows the principles specified in ICHQ3B) | Traits: off-white solid powder; purity: 99.65%; the number of impurities above the reporting limit is 5; total impurities: 0.35% | Traits: off-white solid powder; purity: 99.65%; the number of impurities above the reporting limit is 5; total impurities: 0.35% |
| | | Day 30 | Day 30 |
| | | Traits: off-white solid powder; purity: 99.53%; the number of impurities above the reporting limit is 6; total impurities: 0.47% | Traits: off-white solid powder; purity: 99.60%; the number of impurities above the reporting limit is 6; total impurities: 0.40% |

Table 18-3

| Test sample | Crystal form 2 | | |
|---|---|---|---|
| Sampling time | Day 0, Day 10, Day 30 | | |
| Test item | Traits, purity and total impurities | | |
| Experimental condition | | High temperature (50°C) | Illumination (illuminance: 5000Lx) |
| Experimental result | Day 0 | Day 10 | Day 10 |
| | Traits: off-white solid powder purity: 99.37% the number of impurities above the reporting limit is 7 | Traits: light yellow solid powder purity: 99.20% the number of impurities above the reporting limit is 9 | Traits: light yellow solid powder purity: 99.01% the number of impurities above the reporting limit is 10 |
| | total impurities: 0.63% | total impurities: 0.80% | total impurities: 0.99% |
| | | Day 30 | Day 30 |
| | | Traits: light yellow solid powder; the number of impurities above the reporting limit is 13; purity: 98.72%; total impurities: 1.28% | Traits: light yellow solid powder; the number of impurities above the reporting limit is 14; purity: 98.0%; total impurities: 2.0% |

Table 18-4

<table>
<tr><td>Test sample</td><td colspan="3">Crystal form 3</td></tr>
<tr><td>Sampling time</td><td colspan="3">Day 0, Day 10, Day 30</td></tr>
<tr><td>Test item</td><td colspan="3">Traits, purity and total impurities</td></tr>
<tr><td colspan="2">Experimental condition</td><td>High temperature (50°C)</td><td>Illumination (illuminance: 5000Lx)</td></tr>
<tr><td rowspan="4">Experimental result</td><td>Day 0</td><td>Day 10</td><td>Day 10</td></tr>
<tr><td rowspan="3">Traits: off-white solid powder; purity: 99.44%; the number of impurities above the reporting limit is 7; total impurities: 0.56%</td><td>Traits: light yellow solid powder; purity: 99.28%; the number of impurities above the reporting limit is 7; total impurities: 0.72%</td><td>Traits: light yellow solid powder; the number of impurities above the reporting limit is 10; purity: 98.83%; total impurities: 1.17%</td></tr>
<tr><td>Day 30</td><td>Day 30</td></tr>
<tr><td>Traits: light yellow solid powder; purity: 98.89%; the number of impurities above the reporting limit is 10; total impurities: 1.11%</td><td>Traits: light yellow solid powder; the number of impurities above the reporting limit is 17; purity: 96.95%; total impurities: 3.05%</td></tr>
</table>

Example 15: Solubility test

The samples in fine powder of the compound DA, crystal form 1 (prepared in Examples 1-2), crystal form 2 and crystal form 3 were weighed and added to a certain amount of solvent at 25 °C ±2 °C, shook strongly for 30 seconds every 5 minutes, and the solubility for 24 h was tested.

The solubility test showed that in the solution with a pH of 6.8, the solubility of crystal form 1 was better than that of the amorphous compound DA, crystal form 2 and crystal form 3, as shown in Table 18-5.

Table 18-5

| Sample name | Dissolving Medium | Solubility for 24h (mg/mL) |
|---|---|---|
| Compound DA | Solution with pH=6.8 | 2.11 |
| Crystal form 1 | | 3.26 |
| Crystal form 2 | | 1.36 |
| Crystal form 3 | | 1.23 |

In view of the above three aspects of properties, hygroscopicity, stability and solubility, compared with compound DA, crystal form 1 has better hygroscopicity and solubility, and such an effect is unexpected, and compared with crystal form 2 and crystal form 3, crystal form 1 has better hygroscopicity, stability and solubility, so it is the dominant crystal form.

Example 16: **Preparation of control compounds**

Preparation of control compound 1

Control compound 1A (intermediate):

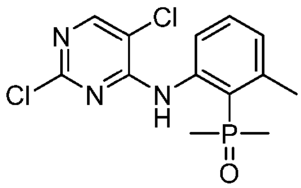

2-amino-6-methylphenyl-dimethyl phosphorus oxide (4.52 g, 24.67 mmol), 2,4,5-trichloropyrimidine (4.98 g, 27.14 mmol), $K_2CO_3$ (4.11 g, 29.60 mmol), and 27 mL of DMSO were added to a three-mouth bottle of 250 mL, and the reaction was heated to 60°C for about 3.5 h until the reaction was completed by TLC detection. The reaction was cooled to room temperature, 90mL of $H_2O$ was added, a large number of yellow solids were precipitated, continually stirred for about 0.5h and then filtered by suction, the filter cake was slurried with 25mL of petroleum ether/ethyl acetate = 4/1 at room temperature for 4h, then filtered by suction, and dried to obtain the compound 1A (6.60 g, yield 81.0%). MS-ESI (m/z): 332.1483$(M+H)^+$.

**Control compound 1:**

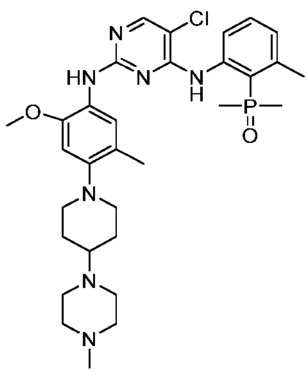

Compound 1A (600 mg, 1.82 mmol), compound 2-methoxy-5-methyl-4-(4-(1-methylpiperidin-4-yl)piperazin-1-yl)aniline (636 mg, 2.00 mmol), 15% hydrogen chloride in ethanol (1.33 g, 5.46 mmol), and 9.0mL of ethylene glycol monomethyl ether were added to the sealed tube, and then the reaction was carried out at 100 °C for 7-8h, after the reaction was completed by TLC detection, the reaction solution was cooled to room temperature, and then 18mL of water and 9.0mL of saturated $NaHCO_3$ aqueous solution were added to precipitate a large number of solids, and after standing for 0.5 h, filtered, the filter cake was washed with water, and after dried, the control compound 1 (500 mg, yield 45.5%) was purified by slurrying with a mixed solvent of 4ml EA and 1.0mL of methanol. $^1H$ NMR (400 MHz, methanol-d4) $\delta$ = 8.02 (s, 1H), 7.93 (dd, J = 8.0, 4.0 Hz, 1H), 7.64 (s, 1H), 7.42 (t, J = 8.0 Hz, 1H), 7.15 (dd, J = 7.6, 3.6 Hz, 1H), 6.69 (s, 1H), 3.84 (s, 3H), 3.11 (d, J = 11.6 Hz, 2H), 3.02 - 2.41 (m, 13H), 2.32 (m, 4H), 2.04 (s, 3H), 2.00 (d, J = 12.6 Hz, 2H), 1.94 (s, 3H), 1.90 (s, 3H), 1.68 (m, 2H). MS-ESI (m/z): 612.2981 $(M+H)^+$.

**Preparation of control compound 2**

**Control compound 2A (intermediate):**

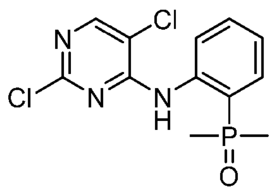

(2-aminophenyl)-dimethylphosphorus oxide (2.5 g, 15.00 mmol), 2,4,5-trichloropyrimidine (2.70 g, 15.00 mmol), $K_2CO_3$ (2.45 g, 67.75 mmol), $nBu_4NHSO_4$ (0.5 g, 1.50 mmol), and 50mL of DMF were added to a 100mL of three-mouth

bottle, and the reaction was heated to 65°C for about 4.5 h until the reaction was completed by TLC detection. Subsequently, the reaction solution was cooled to room temperature, 200mL of $H_2O$ was added, a large number of yellow solids were precipitated, continually stirred for about 0.5h, and then filtered by suction, the filter cake was washed with 100mL of $H_2O$, and dried to obtain the control compound 2A (2.84 g, yield 60.0%). MS-ESI (m/z): 316.0178$(M+H)^+$.

**Control compound 2:**

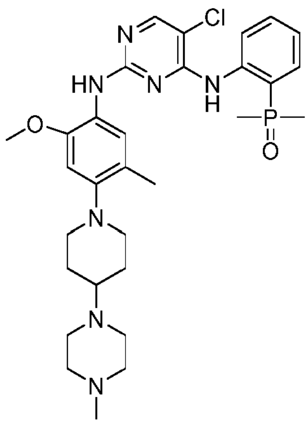

Control compounds 2A (335 mg, 1.06 mmol), 2-methoxy-5-methyl-4-(4-(1-methylpiperidin-4-yl)piperazin-1-yl) aniline (406 mg, 1.27 mmol), 15% hydrogen chloride in ethanol (774 mg, 3.18 mmol), 4.5mL of ethylene glycol monomethyl ether was added to the reaction flask, and then the reaction was sealed at 120 °C for 5 to 6 h, after the reaction was completed by TLC detection, the reaction solution was cooled to room temperature, and then 15mL of saturated $NaHCO_3$ aqueous solution was added, a large number of solids were precipitated, continually stirred for 0.5 h and filtered by suction, the filter cake was washed with water, and the filter cake was slurried with 9 mL of a mixed solvent of $EtOH/H_2O$=1/2 and purified to obtain the control compound 2 (320 mg, yield 50.5%). $^1H$ NMR (400 MHz, CDCl3) $\delta$ = 10.80 (s, 1H), 8.63 (dd, J = 8.4, 4.4 Hz, 1H), 8.10 (s, 1H), 8.04 (s, 1H), 7.51 (m, 1H), 7.38 - 7.26 (m, 2H), 7.13 (m, 1H), 6.63 (s, 1H), 3.86 (s, 3H), 3.16 (d, J = 12.0 Hz, 2H), 2. 61 (m, 9H), 2.32 (s, 3H), 2.18 (s, 3H), 2.06 (m, 2H), 1.96 (m, 2H), 1.87 (s, 3H), 1.83 (s, 3H), 1.72 (m, 2H). MS-ESI (m/z): 598.2826$(M+H)^+$.

**Preparation of control compound 3**

**Control compound 3A (intermediate):**

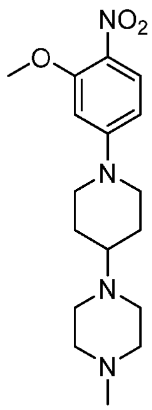

5-fluoro-2-nitroanisole (2.0 g, 11.69 mmol), 1-methyl-4-(4-piperidinyl)piperazine (2.57 g, 14.02 mmol), potassium carbonate (3.25 g, 23.37 mmol), and 30mL of DMF were added to a 100mL of round-bottom flask, and the reaction was heated to 120°C for about 3 h until the reaction was completed by TLC detection. The reaction solution was cooled to

room temperature, added with 30mL of water, extracted with 30 mL ×3 of ethyl acetate, the organic phase was combined, and the organic phase was washed with 30 mL of saturated sodium chloride aqueous solution, dried with anhydrous sodium sulfate, filtered, and concentrated to obtain control compound 3A (3.57 g, yield 91.3%).

**Control compound 3B (intermediate):**

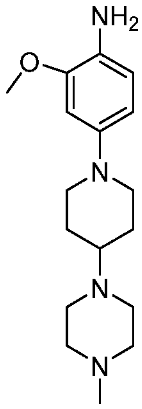

The control compound 3A (3.57 g, 10.68 mmol), 0.36 g of 5% Pd/C, and 72mL of methanol were mixed, and the air therein was replaced with hydrogen gas for 2-3 times, stirred overnight at room temperature in the hydrogen atmosphere (atmospheric pressure) until the reaction was completed by TLC detection, and then filtered through diatomaceous earth, the filtrate was concentrated in vacuum, and isolated and purified by column chromatography to obtain the control compound 3B (2.17 g, yield 66.8%). MS-ESI (m/z): 305.2315$(M+H)^+$.

**Control compound 3C (intermediate):**

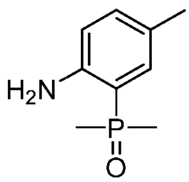

2-iodo-4-methylaniline (10 g, 42.9 mmol), $K_3PO_4$ (10.9 g, 51.5 mmol), Xantphos (2.48 g, 4.3 mmol), $Pd(OAc)_2$ (0.96 g, 4.3 mmol), methylphosphinoylmethane (5 g, 64.4 mmol), and 100mL of DMF were added to a 500mL of three-mouth bottle, and the temperature was raised to 120°C for about 3h until the reaction was completed by TLC detection. The reaction solution was cooled to room temperature, filtered by suction, added with 600mL of $H_2O$, a large number of yellow solids were precipitated, filtered by suction, the filtrate was extracted with 500mL×3 of ethyl acetate, the organic phase was combined, washed with 250mL×2 of saturated sodium chloride aqueous solution, dried with anhydrous sodium sulfate, filtered, and the filtrate was concentrated to obtain compound 3C, which was used in the next step of the reaction without purification. $^1H$ NMR(400MHz, DMSO-d6) δ= 9.21(brs, 2H), 7.57(d, J =13.6, 1H), 7.43(d, J =8.4, 1H), 7.26-7.23(m, 1H), 2.33(s, 3H), 1.85(s, 3H), 1.82(s, 3H).

**Control compound 3D (intermediate):**

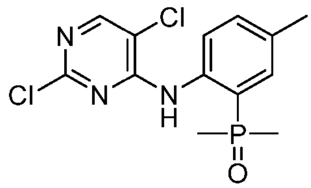

The control compound 3C obtained in the previous step, 2,4,5-trichloropyrimidine (11.80 g, 64.3 mmol), $K_2CO_3$ (7.76 g, 128.73 mmol), $nBu_4NHSO_4$ (1.45 g, 4.29 mmol), and 100mL of DMF were added to a 500mL of three-mouth bottle, and the temperature was raised to 65°C for about 4.5 h until the reaction was completed by TLC detection. The reaction was cooled to room temperature, 200 mL of $H_2O$ was added, a large number of yellow solids were precipitated, continually stirred for about 0.5 h, then filtered by suction, and the filter cake was washed with 100mL of $H_2O$, and dried to obtain the compound 3D (8.57 g, two-step total yield of 60.5%). $^1H$ NMR (400 MHz, DMSO-d6) $\delta$ = 11.62 (s, 1H), 8.40 (s, 1H), 8.27 (dd, J = 8.4, 4.4 Hz, 1H), 7.44 (m, 2H), 2.33 (s, 3H), 1.81 (s, 3H), 1.77 (s, 3H).

**Control compound 3:**

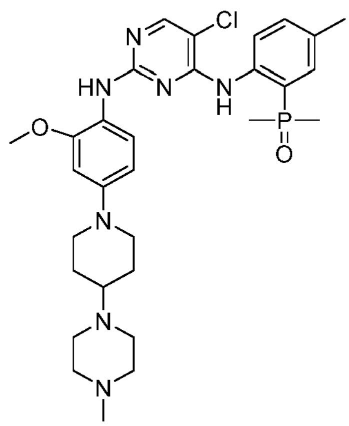

Control compound 3D (400 mg, 1.21 mmol), control compound 3B (443 mg, 1.45 mmol), 15% solution of hydrogen chloride in ethanol (884 mg, 3.63 mmol), and 6mL of ethylene glycol monomethyl ether were added to the reaction flask, and the reaction was sealed at 120°C for 5 to 6 h until the reaction was completed by TLC detection. Subsequently, the reaction solution was cooled to room temperature, added with 6mL of saturated $NaHCO_3$ aqueous solution and 6 mL of $H_2O$, extracted with 30 mL×3 of dichloromethane, the organic phases were combined, washed with 20 mL of aqueous solution of saturated sodium chloride, dried with anhydrous sodium sulfate, filtered, concentrated, purified by column chromatography (DCM/MeOH=8/1), slurried with 5mL of mixed solvent of PE/EA=4/1 to obtain the control compound 3 (257 mg, yield 35.4%). $^1H$ NMR (400 MHz, CDCl3) $\delta$ = 10.63 (s, 1H), 8.49 (dd, J = 8.8, 4.8 Hz, 1H), 8.19 - 7.99 (m, 2H), 7.31 (d, J = 8.8 Hz, 1H), 7.25 (s, 1H), 7.07 (d, J = 14.0 Hz, 1H), 6.61 - 6.44 (m, 2H), 3.87 (s, 3H), 3.66 (d, J = 12.0 Hz, 2H), 2.66 (m, 9H), 2.38 (s, 3H), 2.32 (s, 3H), 2.21 (s, 2H), 1.97 (m, 2H), 1.85 (s, 3H), 1.81 (s, 3H), 1.73 (m, 2H). MS-ESI (m/z): 598.2804$(M+H)^+$.

Example 17: *In vitro* activity experiment of compound DA

Experimental Example 1. Cell anti-proliferation experiment 1

1. Test purpose: to detect the effect of the test compounds on the proliferation of cell lines containing EGFR mutations in BaF3 cells
2. Test information:

2.1. Cell information: Cell lines containing EGFR mutations in BaF3 were from WuXiAppTec:

BaF3-cell lines expressing EGFR mutation

EGFR-T790M/Del19
EGFR-C797S/Del19
EGFR-T790M/L858R
EGFR-C797S/L858R
EGFR-C797S/T790M/L858R
EGFR-C797S/T790M/Del19
EGFR-WT wild-type cell lines

2.2. Test method

Day 0: Cells were seeded in a plate.

a. An ultraviolet lamp of a biological safety cabinet was turned on and countdown was started for 30 minutes.
b. A medium was preheated in a 37°C water bath.
c. After the ultraviolet irradiation was finished, the biological safety cabinet was opened. The pre-heated medium, PBS, and the like, were wiped with alcohol and placed in the biological safety cabinet.
d. The cells were removed from an incubator, blown to a uniform cell suspension in the biological safety cabinet and counted.
e. Based on the cell counting results, the cell suspension was adjusted to a density of 3,000 cells per well and 50 microliters per well, and seeded in 384-well plates.
f. The cells after seeding were incubated in a 5% $CO_2$ incubator at 37°C for 3 hours.
g. Compounds were added to the cell plates using Tecan (liquid workstation).

Day 3: the cell plate added with the compounds and CTG (CellTiter-Glo chemiluminescence cell activity assay reagent) were equilibrated at room temperature, 25 μl of CTG was added to each well, centrifuged at 1,000 rpm for 1 minute, and shaken for 1-2 minutes. And centrifuging for 1 minute at 1,000 rpm again, the cell plates were allowed to settle for 10 minutes to detect a signal value in Envision. The $IC_{50}$ of each compound was calculated by computer fitting with XL-Fit analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/reading value of the control group without drug ×100.

3. Test results: The $IC_{50}$ values for the inhibition activity of the compound DA and the control compounds and the main drugs on the market for the treatment of non-small cell lung cancer, brigatinib and osimertinib, against Ba/F3 cells expressing EGFR-T790M/Del19, EGFR-C797S/Del19, EGFR-T790M/L858R, EGFR-C797S/L858R, EGFR-C797S/T790M/L858R, EGFR-C797S/T790M/Del19 and EGFR-WT are shown in Tables 19 and 20.
4. Conclusion: It can be seen from Tables 19 and 20 that the compound DA has good selectivity for the activity of Ba/F3 cells expressing EGFR wild type (EGFR-WT), and has good inhibitory effect on the proliferation of Ba/F3 cells expressing EGFR double mutation and triple mutation (e.g., EGFR-T790M/Del19, EGFR-C797S/Del19, EGFR-T790M/L858R, EGFR-C797S/L858R, EGFR-C797S/T790M/L858R, EGFR-C797S/T790M/Del19), especially the inhibitory activity on Ba/F3 cell lines expressing EGFR double mutation and triple mutation was much better than that of the control compounds 1, 2 and 3 and the marketed drugs brigatinib and osimertinib.

Table 19

| Tested compound | EGFR-T790M/Del 19 $IC_{50}$(nM) | EGFR-C797S-Del19 $IC_{50}$(nM) | EGFR-T790M/L858R $IC_{50}$ (nM) | EGFR-C797S/L858R $IC_{50}$ (nM) |
|---|---|---|---|---|
| Compound DA | 6.5 | 48.1 | 22.5 | 32.6 |
| Control compound 1 | 165.8 | 184.0 | 156.5 | 572.6 |
| Control compound 2 | 171.8 | 176.6 | - | - |
| Control compound 3 | 216.2 | 202.6 | - | - |
| Brigatinib (Brigatinib) | 164.4 | 177.8 | 148.0 | 547.7 |
| AZD9291 (Osimertinib) | - | 513.4 | - | 1115.0 |

Table 20

| Tested compound | EGFR-C797S/T790M/L858R $IC_{50}$(nM) | EGFR-C797S/T790M/Del19 $IC_{50}$(nM) | EGFR-WT $IC_{50}$ (nM) |
|---|---|---|---|
| Compound DA | 64.1 | 16.5 | 1260.1 |
| Control compound 1 | 626.0 | 180.1 | 1603.4 |
| Control compound 2 | 582.6 | 221.4 | 1760.6 |
| Control compound 3 | 565.8 | 191.7 | 2343.3 |
| Brigatinib (Brigatinib) | 751.6 | 194.3 | 1700.0 |
| AZD9291 (Osimertinib) | 1171.0 | 800.2 | - |

**Experimental Example 2. Cell anti-proliferation experiment 2**

1. Test purpose: to detect the effect of the test compounds on the proliferation of cell lines containing EGFR mutations in PC9 cells
2. Test information:

2.1. Cell information: PC9 cells carried EGFR Del19 mutation, and the rest of mutant cells were constructed from PC9 cells according to the conventional stable cell strain construction method.

PC9-cell lines expressing EGFR mutation
EGFR-Del19
EGFR-T790M/Del19
EGFR-C797S/T790M/Del19

2.2. Test method:

The test compounds were diluted by 5 times each time with an appropriate concentration as an initial concentration of the test, and totally diluted into 6 concentration gradients. Brigatinib (purchased from Selleck) was diluted by three times with 5 $\mu$M as an initial concentration of the test, and totally diluted into 6 concentration gradients. The test compounds and Brigatinib were added into the above cells respectively, and incubated at 37°C and 5%$CO_2$ for 72 hours. SRB (Sulforhodamine B) detection method was used, and the optical density value of each well was read by a microplate reader at 490 nm wavelength.
The optical density of the cells at the drug action of 0 was set as a Tz value representing the value of the cells at the time that the drug was added. The optical density value of the cells after the solvent control DMSO acted for 72 hours was set as a C value. The optical density of the cells on which the test compounds acted for 72 hours was set as a Ti value. The response of the cells to the drug was calculated according to a method proposed by the U.S. NIH-NCI (National Institutes of Health-National Institute of Cancer): When Ti is more than or equal to Tz, the value was [(Ti-Tz)/(C-Tz)] $\times$100; and when Ti is less than Tz, the value was [(Ti-Tz)/Tz] $\times$100. $GI_{50}$ values (the concentration of the test compound required for 50% cell growth inhibition) were calculated according to the above calculation using a 4 Parameter Logistic Model in XLfit software.

3. Test results: The $GI_{50}$ values of compound DA, control compound and brigatinib (the main drug for the treatment of non-small cell lung cancer on the market) on PC9 cells expressing EGFR-Del19, EGFR-T790M/Del19, EGFR-C797S/T790M/Del19, are shown in Table 21.
4. Conclusion: It can be seen from Table 21 that the compound DA has a good inhibitory effect on the proliferation activity of PC9 cells expressing EGFR single, double and triple mutations (such as EGFR-Del19, EGFR-T790M/Del19, EGFR-C797S/T790M/Del19), and the inhibitory activity is much better than that of control compounds 1, 2 and 3 and the marketed drug brigatinib.

Table 21

| Tested compound | EGFR-Del19 $GI_{50}$ (nM) | EGFR-T790M/Del 19 $GI_{50}$ (nM) | EGFR-C797S/T790M/Del19 $GI_{50}$ (nM) |
|---|---|---|---|
| Compound DA | 48.4 | 12.8 | 15.6 |
| Control compound 1 | 265.7 | 267.1 | 336.8 |
| Control compound 2 | 261.6 | 288.9 | 315.8 |
| Control compound 3 | 367.9 | 389.2 | 502.5 |
| Brigatinib (Brigatinib) | 286.9 | 255.8 | 516.1 |

**Experimental Example 3. Cell anti-proliferation experiment** 3

1. Test purpose: to detect the effect of the test compounds on the proliferation of cell lines containing ALK mutations in BaF3 cells
2. Test information:

2.1. Cell information: Cell lines containing ALK mutations in BaF3 were from WuXiAppTec:

Ba/F3-cell lines expressing ALK gene fusion and mutation
Ba/F3-EML-4-ALK-WT
Ba/F3-EML-4-ALK-L1196M

2.2. Test method

Day 0: Cells were seeded in a plate.

a. An ultraviolet lamp of a biological safety cabinet was turned on, and countdown was started for 30 minutes.
b. A medium was preheated in a 37°C water bath.
c. After the ultraviolet irradiation was finished, the biological safety cabinet was opened. The pre-heated medium, PBS, and the like, were wiped with alcohol and placed in the biological safety cabinet.
d. The cells were removed from an incubator, blown to a uniform cell suspension in the biological safety cabinet and counted.
e. Based on the cell counting results, the cell suspension was adjusted to a density of 3,000 cells per well and 50 microliters per well, and seeded in 384-well plates.
f. The cells after seeding were incubated in a 5% $CO_2$ incubator at 37°C for 3 hours.
g. Compounds were added to the cell plates using Tecan (liquid workstation).

Day 3: the cell plate added with the compounds and CTG were equilibrated at room temperature, 25 μl of CTG was added to each well, centrifuged at 1,000 rpm for 1 minute, and shaken for 1-2 minutes. And centrifuging for 1 minute at 1,000 rpm again, the cell plates were allowed to settle for 10 minutes to detect a signal value in Envision. The $IC_{50}$ of each compound was calculated by computer fitting with XL-Fit analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/reading value of the control group without drug $\times$100.

3. Test results: The $IC_{50}$ values of the compound DA and the control compound against BaF3 cells expressing EML-4-ALK-WT and EML-4-ALK-L1196M are shown in Table 22.
4. Conclusion: It can be seen from Table 22 that the compound DA has a very good inhibitory effect on the proliferation of BaF3 cells expressing EML-4-ALK-WT and EML-4-ALK-L1196M, and the inhibitory activity is better than that of the control compounds 1, 2 and 3.

Table 22

| Tested compound | EML-4-ALK-WT $IC_{50}$(nM) | EML-4-ALK-L1196M $IC_{50}$(nM) |
|---|---|---|
| Compound DA | 8.0 | 13.1 |

(continued)

| Tested compound | EML-4-ALK-WT $IC_{50}$(nM) | EML-4-ALK-L1196M $IC_{50}$(nM) |
|---|---|---|
| Control compound 1 | 34.1 | 65.0 |
| Control compound 2 | 43.6 | 47.4 |
| Control compound 3 | 39.4 | 50.2 |

**Experimental Example 4. Cell anti-proliferation experiment 4**

1. Test purpose: to detect the effect of the test compounds on the proliferation of cell lines containing ALK mutations in BaF3 cells
2. Test materials

BaF3 EML-4-ALK-L1196M cell line, brand: KYinno, article number: KC-0102
Cell Counting Kit-8 (CCK-8), brand: Targetmol, article number: C0005
Multifunctional microplate reader, POLARstar Omega, brand: BMG LABTECH

3. Test method

Day 0: Cells were seeded in a plate.

a. The cells were removed from an incubator, blown to a uniform cell suspension in a clean bench and counted.
b. Based on the cell counting results, the cell suspension was adjusted to a density of 8,000 cells per well and 110 μL per well, and plated in a 96-well plate.
c. 10 μL of the compound diluted in gradient was added to the cell plate to give final concentrations of the compounds as 500, 166.67, 55.56, 18.52, 6.17, 2.06, 0.69, 0.23 and 0.08 nM.
d. The cell plate was incubated in a 5% $CO_2$ incubator at 37°C for 72 hours.

Day 3: After placing CCK8 at room temperature for equilibrium, 10 μL of CCK8 was added to each well, the cell plate was incubated in a 5% $CO_2$ incubator at 37°C for 2 hours, and then a signal value $OD_{450}$ was detected in the multifunctional microplate reader POLARstar Omega. The $IC_{50}$ of each compound was calculated by computer fitting with GraphPad prism analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/(reading value of the control group without drug - reading value of the control group with medium only) ×100.

4. Test results: The $IC_{50}$ values of compound DA and compound 1 in the patent application WO2021073498A1 against BaF3 cells expressing EML-4-ALK-L1196M are shown in Table 23.

Conclusion: It can be seen from Table 23 that the compound DA has a very good inhibitory effect on the proliferation of BaF3 cells expressing EML-4-ALK-L1196M, and the inhibitory activity is better than that of the compound 1 in patent application WO2021073498A1.

Table 23: Inhibitory effect on BaF3 EML-4-ALK-L1196M cells of the compound DA and the compound 1 in patent application WO2021073498A1.

| Compound | $IC_{50}$ in the first test (nM) | $IC_{50}$ in the second test (nM) |
|---|---|---|
| Compound DA | 15.14 | 10.38 |
| Compound 1 in patent application WO2021073498A1 | 81.45 | 74.61 |

Example 18: Pharmacodynamic experiment *in vivo* of the crystal form 1 of compound DA

The model used in this trial was an engineered BALB/c nude mouse subcutaneous xenograft model of BaF3

EML-4-ALK-L1196M cells. Suspension culture of engineered BaF3 cells were carried out *in vitro*. BaF3 EML-4-ALK-L1196M was cultured in GIBCO 1640 medium with 10% fetal bovine serum and incubated in a 5% $CO_2$ incubator at 37°C. Cell passage was processed twice a week. When the number of cells reached the requirement, the cells were collected, counted, diluted into a tumor cell suspension with a concentration of $1\times10^7$/ml with PBS, and carried to the animal room in an ice box for direct injection and inoculation. A 1 mL syringe was used to take the above-mentioned prepared tumor cell suspension with a concentration of $1\times10^7$/ml, and the tumor cell suspension was injected subcutaneously into the left forelimb armpit of the dorsal side of the mouse, 0.25 mL for each inoculation site. When the average volume of tumors reached about 160 $mm^3$, the drug was administered for each group, 15 mice in the vehicle group and 10 mice in each of the other administration groups.

Mode and frequency of administration: oral gavage administration, the volume of administration was 10mL/kg, and the model group was given an equal volume of vehicle. It was administered 1 time a day for 14 consecutive days. The dosage of crystal form 1 (prepared in examples 1-2) was 30 mg/kg and 40 mg/kg, and at the same molar dose, the dose of the compound 1 in patent application WO2021073498A1 was 30.2 mg/kg and 40.2 mg/kg.

The mental, active, eating and other general conditions of the mice were observed every day, and the body weight was measured 3 times a week, and the short diameter (a) and long diameter (b) of the tumor of each mouse were measured with vernier calipers 3 times a week, and the tumor volume was calculated according to the formula $(a^2\times b)/2$. The relative tumor volume (RTV) was calculated from the measured calculated tumor volume, RTV = Vt/V0. Wherein V0 was the tumor volume at randomization (i.e., d0) and Vt was the tumor volume at each measurement (i.e., dn). The relative tumor proliferation rate of anti-tumor activity evaluation index was calculated according to the following formula: relative tumor proliferation rate T/C (%):

$$T/C\ \% = \frac{T_{RTV}}{C_{RTV}} \times 100\%$$

(Note: $T_{RTV}$: RTV in the treatment group; $C_{RTV}$: RTV in the model control group. According to the evaluation criteria for efficacy in the "Technical Guidelines for Non-clinical Research of Cytotoxic Antitumor Drugs" issued by the National Medical Products Administration of China, T/C% ≤40% is effective).

Table 24: Mean tumor volume at different time points in each group of the BaF3 EML-4-ALK-L1196M nude mouse xenograft model

| Group | Tumor volume ($mm^3$) | | | | | | |
|---|---|---|---|---|---|---|---|
| | Day 0 a | Day 3 | Day 5 | Day 7 | Day 10 | Day 12 | Day 14 |
| Vehicle | 160 | 366 | 539 | 868 | 1337 | 1581 | 1947 |
| Crystal form 1 (30mg/kg) | 160 | 102 | 89 | 75 | 91 | 109 | 101 |
| Crystal form 1 (40mg/kg) | 159 | 83 | 52 | 49 | 60 | 55 | 49 |
| Compound 1 in patent application WO2021073498A1 (30.2mg/kg) | 161 | 386 | 550 | 789 | 1393 | 1683 | 2547 |
| Compound 1 in patent application WO2021073498A1 (40.2mg/kg) | 159 | 228 | 297 | 512 | 850 | 1279 | 1605 |
| Note: a. Days after administration. | | | | | | | |

Table 25: Relative tumor proliferation rate T/C (%) in the BaF3 EML-4-ALK-L1196M nude mouse xenograft model

| Time / Group | Day 3 T/C(%) | Day 5 T/C(%) | Day 7 T/C(%) | Day 10 T/C(%) | Day 12 T/C(%) | Day 14 T/C(%) |
|---|---|---|---|---|---|---|
| Crystal form 1 (30mg/kg) | 28.53 ▲▲ | 16.41 ▲▲ | 8.23 ▲▲ | 6.61▲▲ | 6.34 ▲▲ | 4.85▲▲ |
| Crystal form 1 (40mg/kg) | 23.88 ▲▲ | 10.09▲▲ | 5.93 ▲▲ | 4.45▲▲ | 3.28 ▲▲ | 2.34▲▲ |
| Compound 1 in patent application WO2021073498 A1 (30.2mg/kg) | 103.90★★ | 103.10** | 90.27 ★★ | 102.75★★ | 103.36★★ | 126.04★★ |

(continued)

| Time<br>Group | Day 3<br>T/C(%) | Day 5<br>T/C(%) | Day 7<br>T/C(%) | Day 10<br>T/C(%) | Day 12<br>T/C(%) | Day 14<br>T/C(%) |
|---|---|---|---|---|---|---|
| Compound 1 in patent application WO2021073498 A1 (40.2mg/kg) | 61.36▲ ▲★★ | 55.09▲▲ ★★ | 58.43 ▲ ▲★★ | 65.08▲ ★★ | 79.27★★ | 80.71★★ |
| A: Comparison of the original RTV value with the RTV value of the vehicle control group, p<0.05, ▲▲: p<0.01; and *: Comparison of the original RTV value with that of the same molar dose of the crystal form 1 group, p<0.05, **: p<0.01. | | | | | | |

The above test results show that:
In this experiment, the crystal form 1 at the dose of 30 mg/kg and 40 mg/kg can significantly inhibit the growth of BaF3 EML-4-ALK-L1196M xenograft tumor, and there is a dose-effect relationship between each dose group. At the end of the D14 experiment, the average tumor volume of tumor-bearing mice in the vehicle control group was 1947 $mm^3$, and the T/C% of crystal form 1 at 30 mg/kg and 40 mg/kg were < 40%, that is, 4.85% and 2.34%, respectively, and the tumor volumes were 101 $mm^3$ and 49 $mm^3$, respectively (compared with the vehicle control group, the p-values were <0.0001 and <0.0001, respectively).

The compound 1 in patent application WO2021073498A1 at the dose of 30.2 mg/kg and 40.2 mg/kg have no significant inhibitory effect on the growth of BaF3 EML-4-ALK-L1196M xenograft tumor. At the end of the D14 experiment, the T/C% thereof was 126.04% and 80.71%, respectively, which did not meet the effective standard, and the tumor volumes were 2547 $mm^3$ and 1605 $mm^3$, respectively.

The crystal form 1 at the doses of 30 mg/kg and 40 mg/kg inhibited the growth of BaF3 EML-4-ALK-L1196M xenograft tumors significantly better than that of the compound 1 in patent application WO2021073498A1 with the same molar dose at the doses of 30.2 mg/kg and 40.2 mg/kg (T/C% of D14: 4.85 % vs. 126.04%, p<0.0001; 2.34% vs. 80.71%, p<0.0001).

## Claims

1. A crystal of dihydrate of (2-((5-bromo-2-((2-methoxy-5-methyl-4-(4-(4-methylpiperazin-1-yl)piperidin-1-yl)phenyl) amino)pyrimidin-4-yl)amino)-5-methylphenyl)dimethylphosphorus oxide with crystal form 1, wherein using Cu-$K_\alpha$ radiation and shown at 2θ angle, the X-ray powder diffraction pattern of the crystal form 1 has the following characteristic peaks: 5.54±0.2°, 11.06°±0.2, 19.12°±0.2 and 27.90±0.2°.

2. The crystal of claim 1 of dihydrate of (2-((5-bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphorus oxide with crystal form 1, wherein using Cu-$K_\alpha$ radiation and shown at 2θ angle, the X-ray powder diffraction pattern of the crystal form 1 has the following characteristic peaks: 5.54±0.2°, 11.06±0.2°, 16.15±0.2°, 16.73±0.2°, 17.10±0.2°, 19.12±0.2°, 21.18±0.2°, 24.16 ±0.2°, 26.27±0.2°, 26.48±0.2°, 27.90±0.2°and 33.64±0.2°.

3. The crystal according to any one of the preceding claims of dihydrate of (2-((5-bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphorus oxide with crystal form 1, wherein using Cu-$K_\alpha$ radiation and shown at 2θ angle, the X-ray powder diffraction pattern of the crystal form 1 has the following characteristic peaks: 5.54±0.2°, 8.50±0.2°, 10.53±0.2°, 11.06±0.2°, 11.48±0.2°, 13.00±0.2°, 16.15±0.2°, 16.73±0.2°, 17.10±0.2°, 19.12±0.2°, 19.47±0.2°, 21.18±0.2°, 24.16±0.2°, 26.27±0.2°, 26.48±0.2°, 27.90±0.2° and 33.64±0.2°.

4. The crystal according to any one of the preceding claims of dihydrate of (2-((5-bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphorus oxide with crystal form 1, wherein using Cu-$K_\alpha$ radiation and shown at 2θ angle, the X-ray powder diffraction pattern of the crystal form 1 has the following characteristic peaks: 5.54±0.2°, 8.50±0.2°, 10.53±0.2°, 11.06±0.2°, 11.48±0.2°, 13.00±0.2°, 16.15±0.2°, 16.73±0.2°, 17.10±0.2°, 18.34±0.2°, 19.12±0.2°, 19.47±0.2°, 21.18±0.2°, 22.21±0.2°, 22.65±0.2°, 23.10±0.2°, 23.49±0.2°, 24.16±0.2°, 26.27±0.2°, 26.48±0.2°, 26.93±0.2°, 27.35±0.2°, 27.90±0.2°, 29.35±0.2°, 32.22±0.2°, 33.64±0.2° and 34.17±0.2°.

5. The crystal according to any one of the preceding claims of dihydrate of (2-((5-bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphorus oxide with crystal form 1, wherein using Cu-$K_{\alpha}$ radiation and shown at 2θ angle, the X-ray powder diffraction pattern of the crystal form 1 is shown in Figure 1.

**6.** A pharmaceutical composition, comprising the crystal of dihydrate according to any one of the preceding claims and a pharmaceutically acceptable carrier.

**7.** A pharmaceutical composition, comprising the crystal of dihydrate according to any one of the preceding claims and other anticancer drug or antitumor drug.

**8.** The pharmaceutical composition of claim 7, wherein the anticancer drug or antitumor drug is one or more of cytotoxic drug, hormone drug, antimetabolite drug, tumor-targeted drug, PARP inhibitor drug, adjuvant therapy drug or antitumor biological drug.

**9.** The pharmaceutical composition of claim 8, wherein the cytotoxic drug is one or more of carboplatin, cisplatin, irinotecan, paclitaxel, fluorouracil, cytarabine, lenalidomide, and tretinoin; the hormone drug is one or more of dexamethasone, fulvestrant, and tamoxifen; the antimetabolite drug is one or more of fluorouracil, methotrexate, furanofluorouracil, and cytarabine; the tumor-targeted drug is one or more of imatinib, erlotinib, and lapatinib; the PARP inhibitor drug is one or more of Olaparib, Rubraca, and Zejula; the adjuvant therapy drug is one or more of the recombinant human granulocyte colony-stimulating factor, erythropoietin, disodium pamidronate, and zoledronic acid; and the antitumor biological drug is one or more of Keytruda, Opdivo, Tecentriq, Imfinzi, and Bavencio.

**10.** The crystal of dihydrate of (2-((5-bromo-2-((2-methoxy-5-methyl-4-(4-(4-methylpiperazin-1-yl)piperidin-1-yl)phenyl) amino)pyrimidin-4-yl)amino)-5-methylphenyl)dimethylphosphorus oxide with crystal form 1 of any one of claims 1-5, the pharmaceutical composition of any one of claims 6-9 for use in preventing and/or treating cancer.

**11.** The crystal of dihydrate of (2-((5-bromo-2-((2-methoxy-5-methyl-4-(4-(4-methylpiperazin-1-yl)piperidin-1-yl)phenyl) amino)pyrimidin-4-yl)amino)-5-methylphenyl)dimethylphosphorus oxide with crystal form 1 of any one of claims 1-5, the pharmaceutical composition of any one of claims 6-9 for use in preventing and/or treating plasmacytoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophagus cancer, squamous cell carcinoma, or colon cancer.

**12.** The crystal of dihydrate of (2-((5-bromo-2-((2-methoxy-5-methyl-4-(4-(4-methylpiperazin-1-yl)piperidin-1-yl)phenyl) amino)pyrimidin-4-yl)amino)-5-methylphenyl)dimethylphosphorus oxide with crystal form 1 of any one of claims 1-5, the pharmaceutical composition of any one of claims 6-9 for use as an EGFR inhibitor or ALK inhibitor or EGFR and ALK inhibitor or protein kinase inhibitor.

**13.** The crystal of dihydrate of (2-((5-bromo-2-((2-methoxy-5-methyl-4-(4-(4-methylpiperazin-1-yl)piperidin-1-yl)phenyl) amino)pyrimidin-4-yl)amino)-5-methylphenyl)dimethylphosphorus oxide with crystal form 1 or the pharmaceutical composition of claim 12, wherein the EGFR inhibitor or ALK inhibitor or EGFR and ALK inhibitor or protein kinase inhibitor is used for the treatment of plasmacytoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophagus cancer, squamous cell carcinoma, or colon cancer; preferably, the EGFR has one or more mutations selected from the group consisting of L858R mutation, Del19 mutation, T790M mutation and C797S mutation; and further preferably, the EGFR has C797S mutation; and
preferably, the ALK has EML-4-ALK fusion and/or EML-4-ALK-L1196M mutation.

## Patentansprüche

**1.** Kristall eines Dihydrats von (2-((5-Brom-2-((2-methoxy-5-methyl-4-(4-(4-methylpiperazin-1-yl)piperidin-1-yl)phenyl) amino)pyrimidin-4-yl)amino)-5-methylphenyl)dimethylphosphoroxid mit einer Kristallform 1, wobei bei einer Verwendung einer Cu-$K\alpha$-Strahlung und bei einem 2θ-Winkel gezeigt das Röntgenstrahl-Pulverdiffraktionsmuster der Kristallform 1 die folgenden charakteristischen Spitzen aufweist: 5,54±0,2°, 11,06°±0,2, 19,12°±0.2 und 27,90 ±0,2°.

2. Kristall nach Anspruch 1 eines Dihydrats von (2-((5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1, wobei bei einer Verwendung einer Cu-$K\alpha$-Strahlung und bei einem 2θ-Winkel gezeigt das Röntgenstrahl-Pulverdiffraktionsmuster der Kristallform 1 die folgenden charakteristischen Spitzen aufweist: 5,54±0m2°, 11,06±0,2°, 16,15±0,2°, 16,73±0,2°, 17,10±0,2°, 19,12±0,2°, 21,18±0,2°, 24,16±0,2°, 26,27±0,2°, 26,48±0,2°, 27,90±0,2° und 33,64±0,2°.

3. Kristall nach einem der vorstehenden Ansprüche eines Dihydrats von (2-(5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1, wobei bei einer Verwendung einer Cu-$K\alpha$-Strahlung und bei einem 20-Winkel gezeigt das Röntgenstrahl-Pulverdiffraktionsmuster der Kristallform 1 die folgenden charakteristischen Spitzen aufweist: 5,54 ±0,2°, 8,50±0,2°, 10,53±0,2°, 11,06±0,2°, 11,48±0,2°, 13,00±0,2°, 16,15±0,2°, 16,73±0,2°, 17,10±0,2°, 19,12 ±0,2°, 19,47±0,2°, 21,18±0,2°, 24,16±0,2°, 26,27±0,2°, 26,48±0,2°, 27,90±0,2° und 33,64±0,2°.

4. Kristall nach einem der vorstehenden Ansprüche eines Dihydrats von (2-(5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1, wobei bei einer Verwendung einer Cu-$K\alpha$-Strahlung und bei einem 20-Winkel gezeigt das Röntgenstrahl-Pulverdiffraktionsmuster der Kristallform 1 die folgenden charakteristischen Spitzen aufweist: 5,54 ±0,2°, 8,50±0,2°, 10,53±0,2°, 11,06±0,2°, 11,48±0,2°, 13,00±0,2°, 16,15±0,2°, 16,73±0,2°, 17,10±0,2°, 18,34 ±0,2°, 19,12±0,2°, 19,47±0,2°, 21,18±0,2°, 22,21±0,2°, 22,65±0,2°, 23,10±0,2°, 23,49±0,2°, 24,16±0,2°, 26,27 ±0,2°, 26,48±0,2°, 26,93±0,2°, 27,35±0,2°, 27,90±0,2°, 29,35±0,2°, 32,22±0,2°, 33,64±0,2° und 34,17±0,2°.

5. Kristall nach einem der vorstehenden Ansprüche eines Dihydrats von (2-((5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1, wobei bei einer Verwendung einer Cu-$K\alpha$-Strahlung und bei einem 20-Winkel gezeigt das Röntgenstrahl-Pulverdiffraktionsmuster der Kristallform 1 das in Fig. 1 gezeigte ist.

6. Pharmazeutische Zusammensetzung, die den Kristall eines Dihydrats gemäß einem der vorstehenden Ansprüche und einen pharmazeutisch annehmbaren Träger umfasst.

7. Pharmazeutische Zusammensetzung, die den Kristall eines Dihydrats gemäß einem der vorstehenden Ansprüche und ein weiteres Antikrebsmittel oder Antitumormittel umfasst.

8. Pharmazeutische Zusammensetzung nach Anspruch 7, wobei das Antikrebsmittel oder Antitumormittel ein zytotoxisches Mittel, ein Hormonmittel, ein Antimetabolitmittel, ein auf einen Tumor abzielendes Mittel, ein PARP-Inhibitormittel, ein Mittel für eine unterstützende Therapie und/oder ein biologisches Antitumormittel ist.

9. Pharmazeutische Zusammensetzung nach Anspruch 8, wobei das zytotoxische Mittel Carboplatin, Cisplatin, Irinotecan, Paclitaxel, Fluorouracil, Cytarabin, Lenalidomid und/oder Tretinoin ist, wobei das Hormonmittel Dexamethason, Fulvestrant und/oder Tamoxifen ist, wobei das Antimetabolitmittel Fluorouracil, Methotrexat, Furanofluorouracil und/oder Cytarabin ist, wobei das auf einen Tumor abzielende Mittel Imatinib, Erlotinib und/oder Lapatinib ist, wobei das PARP-Inhibitormittel Olaparib, Rubraca und/oder Zejula ist, wobei das Mittel für eine unterstützende Therapie der rekombinante humane Granulozyten-Kolonie-stimulierende Faktor, Erythropoietin, Dinatriumpamidronat und/oder Zoledronsäure ist und wobei das biologische Antitumormittel Keytruda, Opdivo, Tecentriq, Imfinzi und/oder Bavencio ist.

10. Kristall eines Dihydrats von (2-((5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1 nach einem der Ansprüche 1-5 oder pharmazeutische Zusammensetzung nach einem der Ansprüche 6-9 für die Verwendung bei der Prävention und/oder Behandlung von Krebs.

11. Kristall eines Dihydrats von (2-((5-Brom-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1 nach einem der Ansprüche 1-5 oder pharmazeutische Zusammensetzung nach einem der Ansprüche 6-9 für die Verwendung bei der Prävention und/oder Behandlung eines Plasmazytoms, eines Mantelzelltumors, eines multiplen Myeloms, eines Melanoms, eines Brustkrebs, eines Leberkrebs, eines Gebärmutterhalskrebs, eine Lungenkrebs, eines Lymphoms, einer Leukämie, eines Eierstockkrebs, eines Nierenkrebs, eines Magenkrebs, eines Nasopharynxkrebs, eines Schilddrüsenkrebs, eines Bauchspeicheldrüsenkrebs, eines Prostatakrebs, eines A-denokarzinoms, eines Mundkrebs, eines Speiseröhrenkrebs, eines Plattenepithelkarzinoms oder eines Darmkrebs.

**12.** Kristall eines Dihydrats von (2-((5-Bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1 nach einem der Ansprüche 1-5 oder pharmazeutische Zusammensetzung nach einem der Ansprüche 6-9 für die Verwendung als ein EGFR-Inhibitor oder ALK-Inhibitor oder ein EGFR- und ALK-Inhibitor oder ein Proteinkinaseinhibitor.

**13.** Kristall eines Dihydrats von (2-((5-Bromo-2-((2-methoxy-5-methyl-4-(4-(4-methylpiperazin-1-yl)piperidin-1-yl)phenyl)amino)pyrimidin-4-yl)amino)-5-methylphenyl)dimethylphosphoroxid mit einer Kristallform 1 oder pharmazeutische Zusammensetzung nach Anspruch 12, wobei der EGFR-Inhibitor oder ALK-Inhibitor oder EGFR- und ALK-Inhibitor oder Proteinkinaseinhibitor für die Behandlung eines Plasmazytoms, eines Mantelzelltumors, eines multiplen Myeloms, eines Melanoms, eines Brustkrebs, eines Leberkrebs, eines Gebärmutterkrebs, eines Lungenkrebs, eines Lymphoms, einer Leukämie, eines Eierstockkrebs, eines Nierenkrebs, eines Magenkrebs, eines Nasopharynxkrebs, eines Schilddrüsenkrebs, eines Bauchspeicheldrüsenkrebs, eines Prostatakrebs, eines A-denocarzinoms, eines Mundkrebs, eines Speiseröhrenkrebs, eines Plattenephithelkarzinoms oder eines Darmkrebs verwendet wird, wobei vorzugsweise der EGFR eine oder mehrere Mutationen auweist, die aus der Gruppe ausgewählt sind, die besteht aus einer L858R-Mutation, einer Del19-Mutation, einer T790M-Mutation und einer C797S-Mutation, und wobei vorzugsweise weiterhin der EGFR eine C797S-Mutation aufweist, und wobei vorzugsweise die ALK eine EML-4-ALK-Fusion und/oder eine EML-4-ALK-L1196M-Mutation aufweist.

**Revendications**

**1.** Cristal de dihydrate d'oxyde de (2-((5-bromo-2-((2-méthoxy-5-méthyl-4-(4-(4-méthylpipérazin-1-yl)pipéridin-1-yl) phényl)amino)pyrimidin-4-yl)amino)-5-méthylphényl)diméthylphosphore avec la forme cristalline 1, dans lequel en utilisant le rayonnement Cu-$K_\alpha$ et représenté en angle 2θ, le diagramme de diffraction X de poudre de la forme cristalline 1 présente les pics caractéristiques suivants : 5,54 ± 0.2°, 11,06° ± 0,2, 19,12° ± 0,2 et 27,90 ± 0,2°.

**2.** Cristal selon la revendication 1 de dihydrate d'oxyde de (2-((5-bromo-2-((2-méthoxy-5-méthyl-4-(4-(4-méthylpipérazin-1-yl)pipéridin-1-yl)phényl)amino)pyrimidin-4-yl)amino)-5-méthylphényl)diméthylphosphore avec la forme cristalline 1, dans lequel en utilisant le rayonnement Cu-$K_\alpha$ et représenté en angle 2θ, le diagramme de diffraction X de poudre de la forme cristalline 1 présente les pics caractéristiques suivants : 5,54 ± 0,2°, 11,06 ± 0,2°, 16,15 ± 0,2°, 16,73 ± 0,2°, 17,10 ± 0,2°, 19,12 ± 0,2°, 21,18 ± 0,2°, 24,16 ± 0,2°, 26,27 ± 0,2°, 26,48 ± 0,2°, 27,90 ± 0,2°et 33,64 ± 0,2°.

**3.** Cristal selon l'une quelconque des revendications précédentes de dihydrate d'oxyde de (2-((5-bromo-2-((2-méthoxy-5-méthyl-4-(4-(4-méthylpipérazin-1-yl)pipéridin-1-yl)phényl)amino)pyrimidin-4-yl)amino)-5-méthylphényl)diméthylphosphore avec la forme cristalline 1, dans lequel en utilisant le rayonnement Cu-$K_\alpha$ et représenté en angle 20, le diagramme de diffraction X de poudre de la forme cristalline 1 présente les pics caractéristiques suivants : 5,54 ± 0,2°, 8,50 ± 0,2°, 10,53 ± 0,2°, 11,06 ± 0,2°, 11,48 ± 0,2°, 13,00 ± 0,2°, 16,15 ± 0,2°, 16,73 ± 0,2°, 17,10 ± 0,2°, 19,12 ± 0,2°, 19,47 ± 0,2°, 21,18 ± 0,2°, 24,16 ± 0,2°, 26,27 ± 0,2°, 26,48 ± 0,2°, 27,90 ± 0,2° et 33,64 ± 0,2°.

**4.** Cristal selon l'une quelconque des revendications précédentes de dihydrate d'oxyde de (2-((5-bromo-2-((2-méthoxy-5-méthyl-4-(4-(4-méthylpipérazin-1-yl)pipéridin-1-yl)phényl)amino)pyrimidin-4-yl)amino)-5-méthylphényl)diméthylphosphore avec la forme cristalline 1, dans lequel en utilisant le rayonnement Cu-$K_\alpha$ et représenté en angle 2θ, le diagramme de diffraction X de poudre de la forme cristalline 1 présente les pics caractéristiques suivants : 5,54 ± 0,2°, 8,50 ± 0,2°, 10,53 ± 0,2°, 11,06 ± 0,2°, 11,48 ± 0,2°, 13,00 ± 0,2°, 16,15 ± 0,2°, 16,73 ± 0,2°, 17,10 ± 0,2°, 18,34 ± 0,2°, 19,12 ± 0,2°, 19,47 ± 0,2°, 21,18 ± 0,2°, 22,21 ± 0,2°, 22,65 ± 0,2°, 23,10 ± 0,2°, 23,49 ± 0,2°, 24,16 ± 0,2°, 26,27 ± 0,2°, 26,48 ± 0,2°, 26,93 ± 0,2°, 27,35 ± 0,2°, 27,90 ± 0,2°, 29,35 ± 0,2°, 32,22 ± 0,2°, 33,64 ± 0,2° et 34,17 ± 0,2°.

**5.** - Cristal selon l'une quelconque des revendications précédentes de dihydrate d'oxyde de (2-((5-bromo-2-((2-méthoxy-5-méthyl-4-(4-(4-méthylpipérazin-1-yl)pipéridin-1-yl)phényl)amino)pyrimidin-4-yl)amino)-5-méthylphényl) diméthylphosphore avec la forme cristalline 1, dans lequel en utilisant le rayonnement Cu-$K_\alpha$ et représenté en angle 2θ, le diagramme de diffraction X de poudre de la forme cristalline 1 est illustré sur la Figure 1.

**6.** Composition pharmaceutique, comprenant le cristal de dihydrate selon l'une quelconque des revendications précédentes et un véhicule pharmaceutiquement acceptable.

**7.** Composition pharmaceutique, comprenant le cristal de dihydrate selon l'une quelconque des revendications précédentes et un autre médicament anticancéreux ou médicament antitumoral.

**8.** Composition pharmaceutique selon la revendication 7, dans laquelle le médicament anticancéreux ou médicament antitumoral est un ou plusieurs parmi un médicament cytotoxique, un médicament hormonal, un médicament antimétabolite, un médicament ciblant une tumeur, un médicament inhibiteur de PARP, un médicament de thérapie adjuvante ou un médicament antitumoral biologique.

**9.** Composition pharmaceutique selon la revendication 8, dans laquelle le médicament cytotoxique est un ou plusieurs parmi le carboplatine, le cisplatine, l'irinotécan, le paclitaxel, le fluorouracile, la cytarabine, le lénalidomide et la trétinoïne ; le médicament hormonal est un ou plusieurs parmi la dexaméthasone, le fulvestrant et le tamoxifène ; le médicament antimétabolite est un plusieurs parmi le fluorouracile, le méthotrexate, le furanofluorouracile et la cytarabine ; le médicament ciblant une tumeur est un ou plusieurs parmi l'imatinib, l'erlotinib et le lapatinib ; le médicament inhibiteur de PARP est un ou plusieurs parmi Olaparib, Rubraca et Zejula ; le médicament de thérapie adjuvante est un ou plusieurs parmi le facteur stimulant les colonies de granulocytes humain recombinant, l'érythropoïétine, le pamidronate disodique et l'acide zolédronique ; et le médicament antitumoral biologique est un ou plusieurs parmi Keytruda, Opdivo, Tecentriq, Imfinzi et Bavencio.

**10.** Cristal de dihydrate d'oxyde de (2-(5-bromo-2-(2-méthoxy-5-méthyl-4-(4-(4-méthylpipérazin-1-yl)pipéridin-1-yl)phényl)amino)pyrimidin-4-yl)amino)-5-méthylphényl)diméthylphosphore avec la forme cristalline 1 selon l'une quelconque des revendications 1 à 5, composition pharmaceutique selon l'une quelconque des revendications 6 à 9 pour une utilisation dans la prévention et/ou le traitement du cancer.

**11.** Cristal de dihydrate d'oxyde de (2-((5-bromo-2-((2-méthoxy-5-méthyl-4-(4-(4-méthylpipérazin-1-yl)pipéridin-1-yl) phényl)amino)pyrimidin-4-yl)amino)-5-méthylphényl)diméthylphosphore avec la forme cristalline 1 selon l'une quelconque des revendications 1 à 5, composition pharmaceutique selon l'une quelconque des revendications 6 à 9 pour une utilisation dans la prévention et/ou le traitement du plasmacytome, de la tumeur à cellules du manteau, du myélome multiple, du mélanome, du cancer du sein, du cancer du foie, du cancer du col de l'utérus, du cancer du poumon, du lymphome, de la leucémie, du cancer de l'ovaire, du cancer du rein, du cancer de l'estomac, du cancer du nasopharynx, du cancer de la thyroïde, du cancer du pancréas, du cancer de la prostate, de l'adénocarcinome, du cancer de la bouche, du cancer de l'esophage, du carcinome à cellules squameuses ou du cancer du côlon.

**12.** Cristal de dihydrate d'oxyde de (2-(5-bromo-2-(2-méthoxy-5-méthyl-4-(4-(4-méthylpipérazin-1-yl)pipéridin-1-yl)phényl)amino)pyrimidin-4-yl)amino)-5-méthylphényl)diméthylphosphore avec la forme cristalline 1 selon l'une quelconque des revendications 1 à 5, composition pharmaceutique selon l'une quelconque des revendications 6 à 9 pour une utilisation comme inhibiteur d'EGFR ou inhibiteur d'ALK ou inhibiteur d'EGFR et d'ALK ou inhibiteur de protéine kinase.

**13.** Cristal de dihydrate d'oxyde de (2-((5-bromo-2-((2-méthoxy-5-méthyl-4-(4-(4-méthylpipérazin-1-yl)pipéridin-1-yl) phényl)amino)pyrimidin-4-yl)amino)-5-méthylphényl)diméthylphosphore avec la forme cristalline 1 ou composition pharmaceutique selon la revendication 12, dans lequel l'inhibiteur d'EGFR ou l'inhibiteur d'ALK ou l'inhibiteur d'EGFR et d'ALK ou l'inhibiteur de protéine kinase est utilisé dans le traitement du plasmacytome, de la tumeur à cellules du manteau, du myélome multiple, du mélanome, du cancer du sein, du cancer du foie, du cancer du col de l'utérus, du cancer du poumon, du lymphome, de la leucémie, du cancer de l'ovaire, du cancer du rein, du cancer de l'estomac, du cancer du nasopharynx, du cancer de la thyroïde, du cancer du pancréas, du cancer de la prostate, de l'adénocarcinome, du cancer de la bouche, du cancer de l'œsophage, du carcinome à cellules squameuses ou du cancer du côlon ; de préférence, l'EGFR présente une ou plusieurs mutations choisies dans le groupe constitué par une mutation L858R, une mutation Del19, une mutation T790M et une mutation C797S ; et en outre de préférence, l'EGFR présente une mutation C797S ; et
de préférence, l'ALK présente une fusion EML-4-ALK et/ou une mutation EML-4-ALK-L1196M.

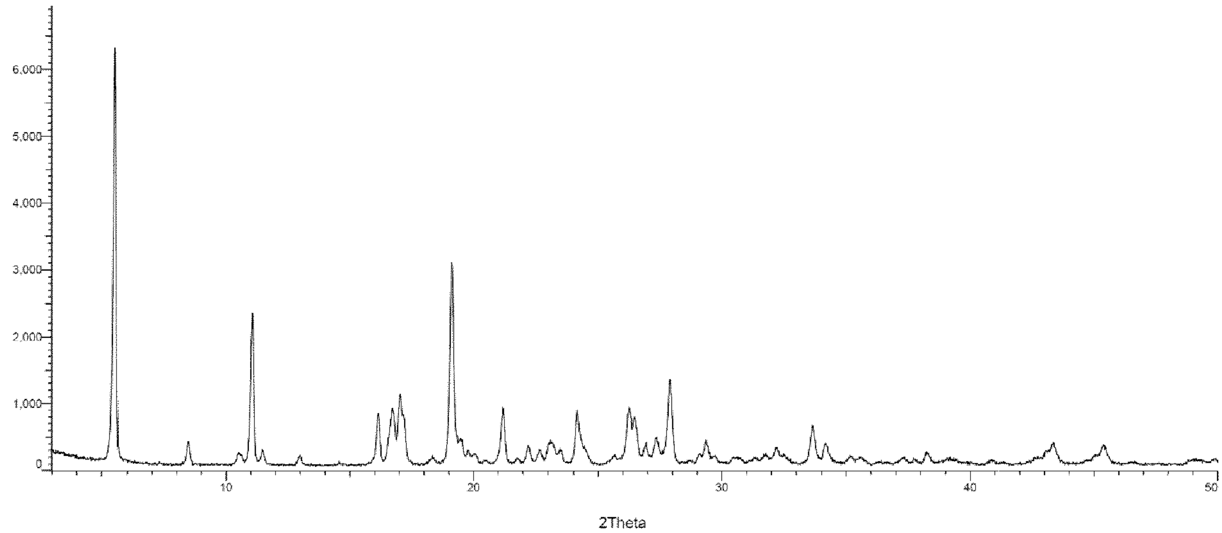
6,000
5,000
4,000
3,000
2,000
1,000
0
10
20
30
40
50
2Theta

Fig. 1

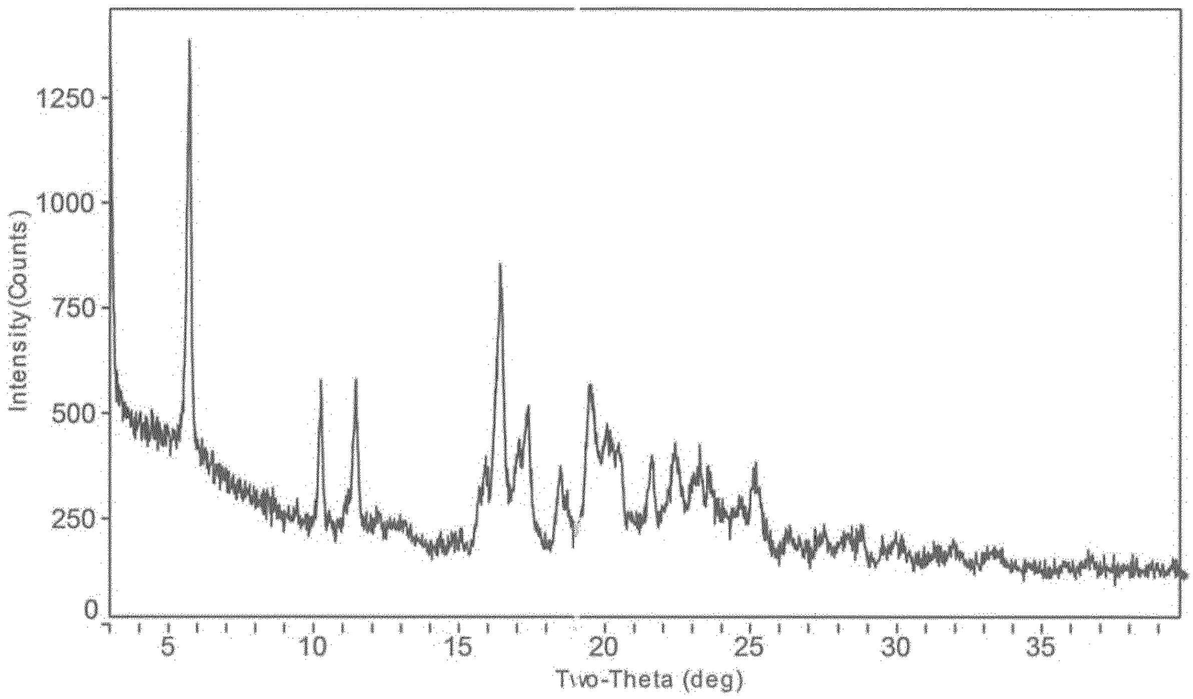
1250
1000
750
500
250
0
Intensity(Counts)
5
10
15
20
25
30
35
Two-Theta (deg)

Fig. 2

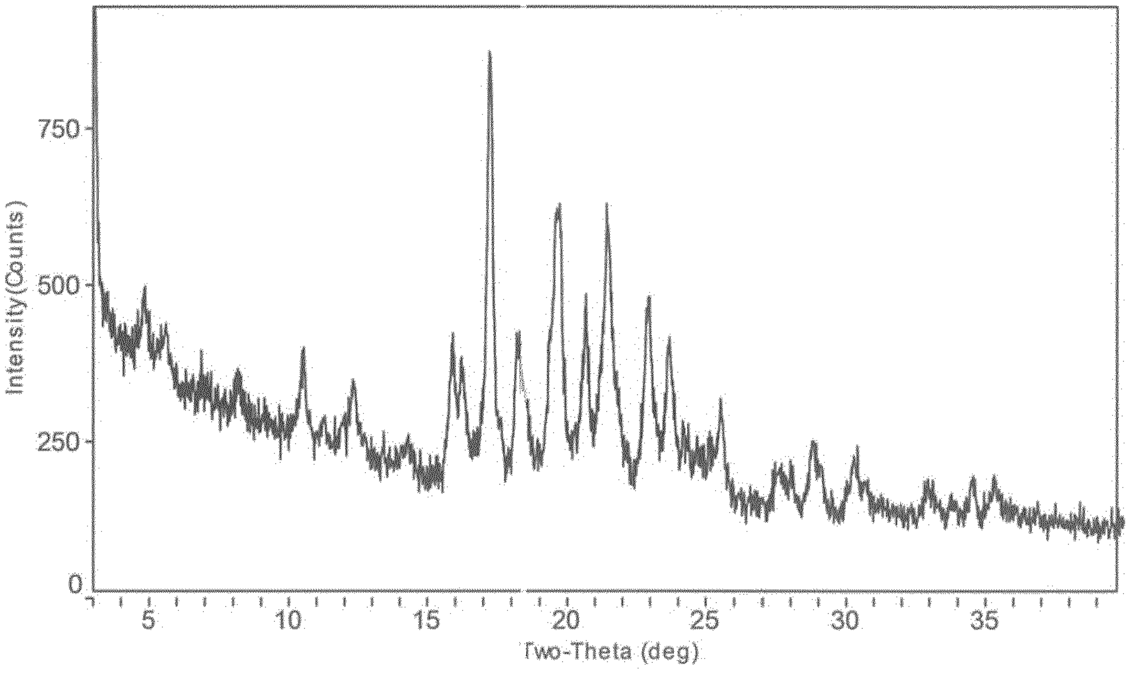


Fig. 3

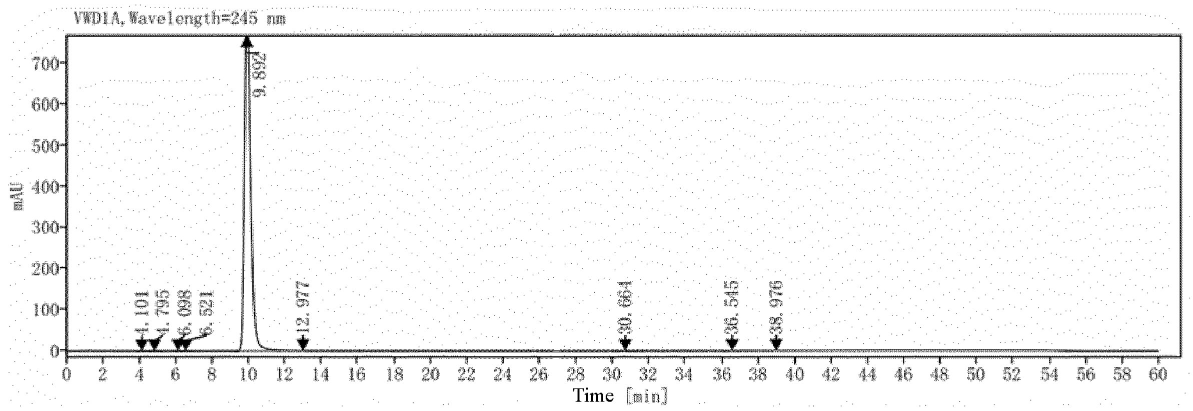


Signal: VWD1A,Wavelength=245 nm

| Compound Name | Retention Time | Relative to Retention Time | Peak Area | Peak Area% | Height | Separation Degree | Trailing Factor | Theoretical Plate Number |
|---|---|---|---|---|---|---|---|---|
| | 4.101 | | 1.667 | 0.01 | 0.1 | | 1.14 | 1733 |
| | 4.795 | | 9.470 | 0.05 | 0.5 | 1.61 | 1.13 | 1691 |
| | 6.098 | | 12.599 | 0.06 | 0.7 | 2.61 | 0.95 | 2103 |
| | 6.521 | | 7.418 | 0.04 | 0.4 | 0.74 | 1.59 | 1795 |
| DA | 9.892 | | 19454.746 | 99.66 | 843.1 | 5.59 | 1.39 | 4500 |
| | 12.977 | | 6.245 | 0.03 | 0.3 | 5.36 | 0.94 | 8514 |
| | 30.664 | | 8.845 | 0.05 | 0.3 | 27.13 | 1.07 | 27386 |
| | 36.545 | | 8.399 | 0.04 | 0.4 | 9.09 | 1.12 | 69720 |
| | 38.976 | | 11.060 | 0.06 | 0.7 | 4.96 | 1.14 | 133781 |

Fig. 4

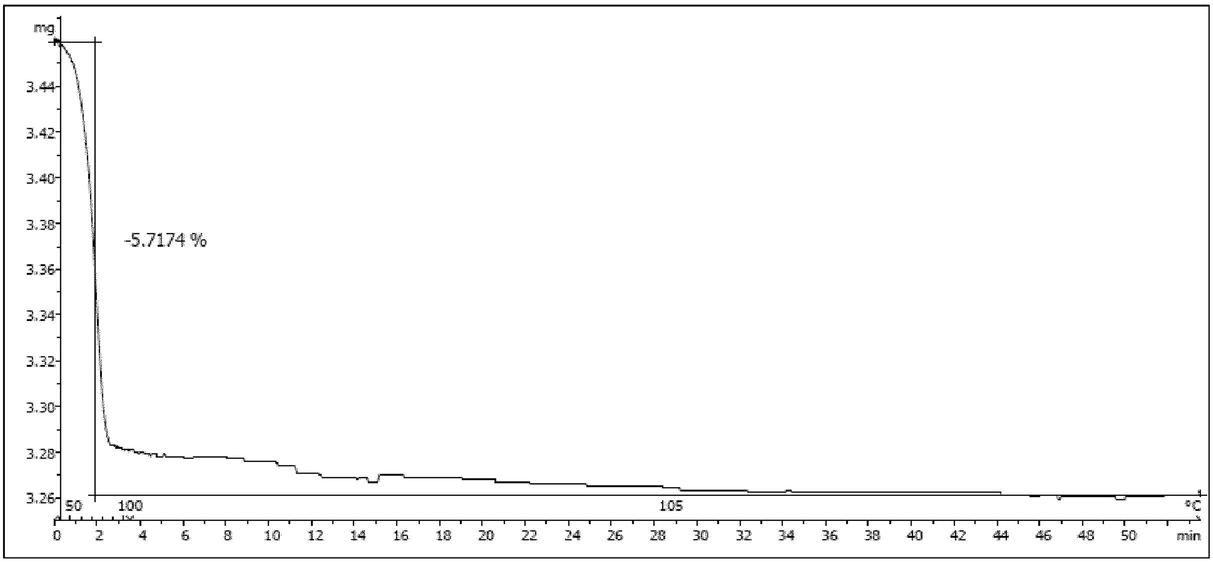
mg
3.44
3.42
3.40
3.38
3.36
3.34
3.32
3.30
3.28
3.26
-5.7174 %
50
100
105
°C
0 2 4 6 8 10 12 14 16 18 20 22 24 26 28 30 32 34 36 38 40 42 44 46 48 50 min

Fig. 5a

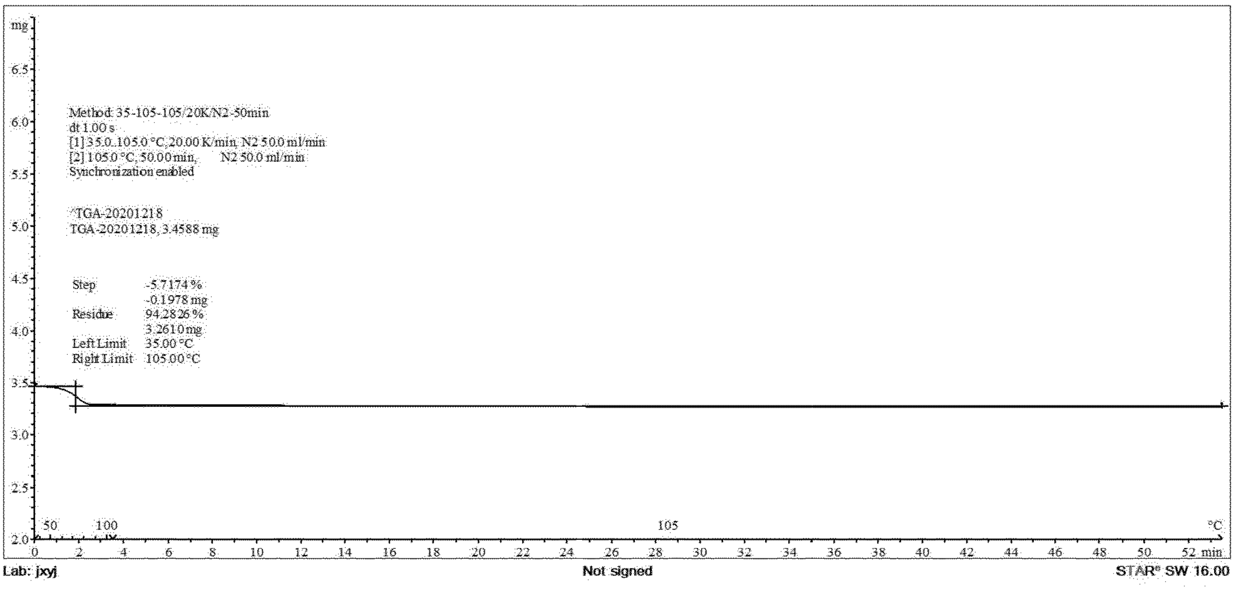
mg
6.5
6.0
5.5
5.0
4.5
4.0
3.5
3.0
2.5
2.0
Method: 35-105-105/20K/N2-50min
dt 1.00 s
[1] 35.0..105.0 °C, 20.00 K/min, N2 50.0 ml/min
[2] 105.0 °C, 50.00 min, N2 50.0 ml/min
Synchronization enabled
TGA-20201218
TGA-20201218, 3.4588 mg
Step -5.7174 %
-0.1978 mg
Residue 94.2826 %
3.2610 mg
Left Limit 35.00 °C
Right Limit 105.00 °C
50
100
105
°C
0 2 4 6 8 10 12 14 16 18 20 22 24 26 28 30 32 34 36 38 40 42 44 46 48 50 52 min
Lab: jxyj
Not signed
STAR® SW 16.00

Fig. 5b

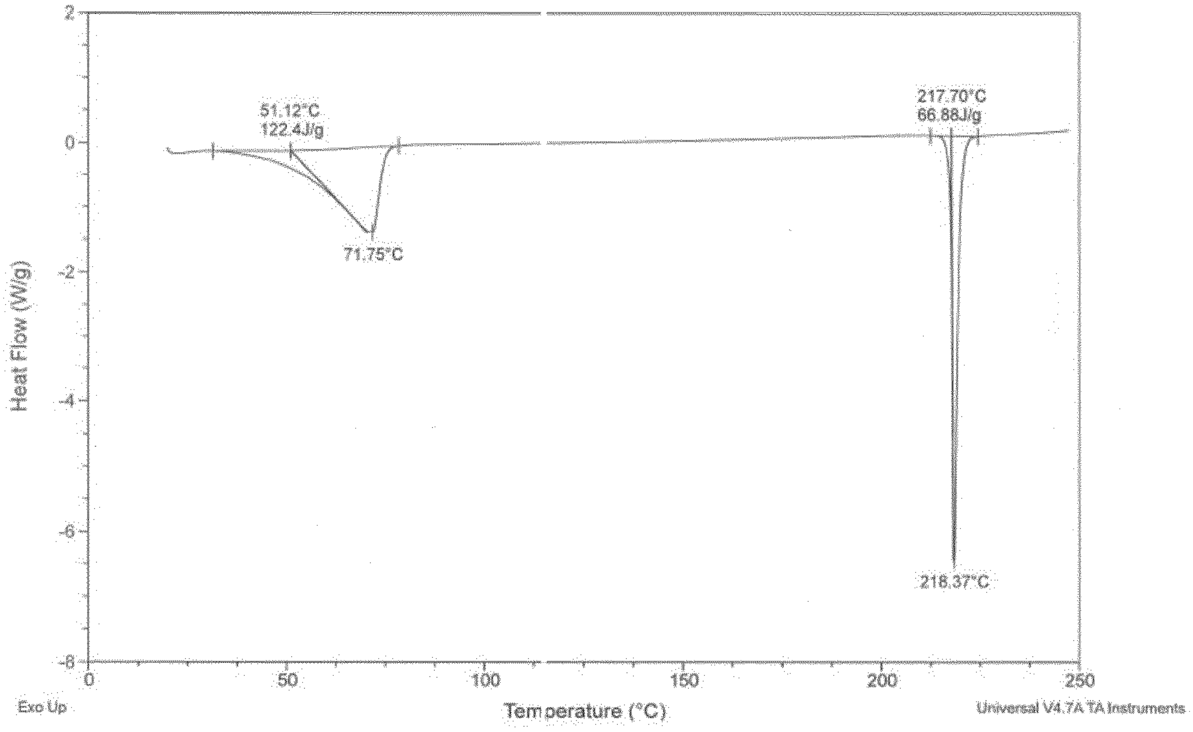
51.12°C
122.4J/g
71.75°C
217.70°C
66.88J/g
218.37°C
Heat Flow (W/g)
Temperature (°C)
Exo Up
Universal V4.7A TA Instruments

**Fig. 6**

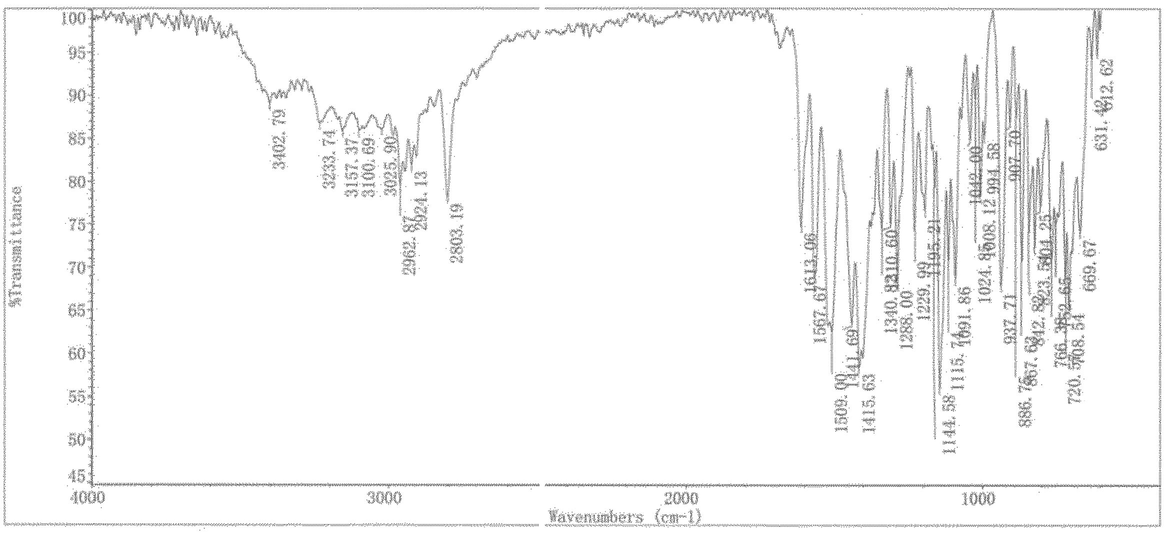
%Transmittance
Wavenumbers (cm-1)
3402.79
3233.74
3157.37
3100.69
3025.90
2962.87
2924.13
2803.19
1613.06
1567.67
1509.00
1441.69
1415.63
1340.82
1310.60
1288.00
1229.99
1195.21
1144.58
1115.74
1091.86
1042.00
1024.85
1008.12
994.58
937.71
907.70
886.76
867.62
842.82
823.54
804.25
766.35
720.57
708.54
669.67
631.49

**Fig. 7**

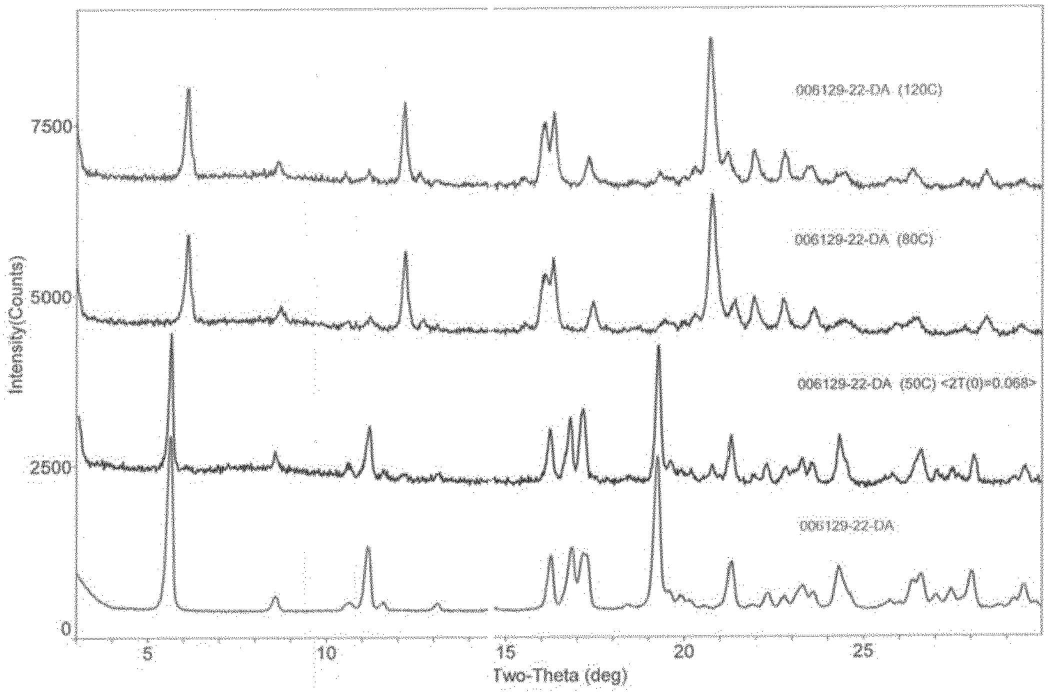
006129-22-DA (120C)
006129-22-DA (80C)
006129-22-DA (50C) <2T(0)=0.068>
006129-22-DA
Intensity(Counts)
7500
5000
2500
0
5
10
15
20
25
Two-Theta (deg)

Fig. 8

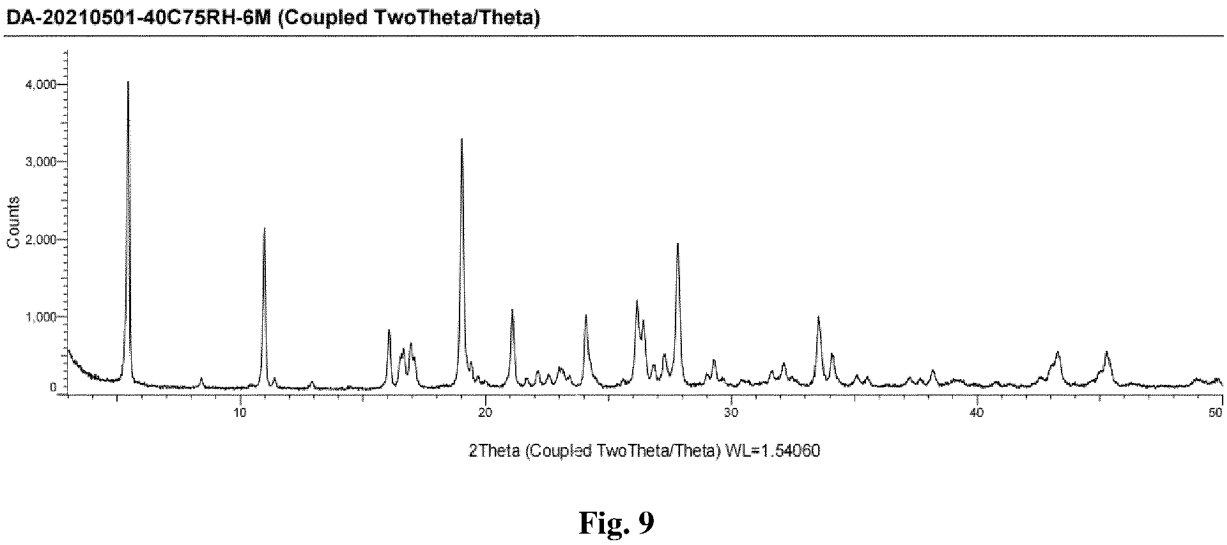
DA-20210501-40C75RH-6M (Coupled TwoTheta/Theta)
Counts
4,000
3,000
2,000
1,000
0
10
20
30
40
50
2Theta (Coupled TwoTheta/Theta) WL=1.54060

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020147838 A1
- WO 2021073498 A1


### Non-patent literature cited in the description


- *Chinese Journal of Lung Cancer [J*, 20 February 2012, vol. 15 (2), 106-111
- *Nature Medicine*, 2015, vol. 21 (6), 560-562
- General Principles 0451 of Part IV of the Chinese Pharmacopoeia. 2020
- General Principles 0832 of Part IV of the Chinese Pharmacopoeia. 2020
- Guiding Principles for the Hygroscopicity of Drugs. Part IV of the Chinese Pharmacopoeia. 2020